# EUROPEAN PATENT APPLICATION

(11) **EP 4 710 927 A1**
(43) Date of publication of application: **18.03.2026**
(21) Application number: 24200310.1
(22) Date of filing: 13.09.2024
(51) Int. Cl.: A61K 31/685, A61K 9/16, A61P 33/02

(54) **OLEYLPHOSPHOCHOLINE IN THE TREATMENT OF LEISHMANIASIS**

(71) Applicant: Oblita Therapeutics BVBA, 2980 Zoersel (BE)
(72) Inventor: Jansen, Caroline, 2980 Zoersel (BE)
(74) Representative: Nederlandsch Octrooibureau

(57) **Abstract**

The present invention provides therapeutic and/or prophylactic treatments against leishmaniasis in non-human mammals in need of such treatment, said treatments comprising administering oleyl phosphocholine to said non-human mammal. The invention also relates to veterinary (pharmaceutical) compositions adapted, suitable and/or intended for use in these treatments.

## Description

### Field of the Invention

The present invention relates to the fields of veterinary medicine and parasitology. In particular, the present invention provides therapeutic and/or prophylactic treatments against leishmaniasis. The invention also relates to veterinary (pharmaceutical) compositions adapted, suitable and/or intended for use in these treatments.

### Background of the Invention

Visceral leishmaniasis caused by the protozoan *Leishmania* is a life threatening parasitic disease occurring in humans as well as other mammals. *Leishmania* parasites are transmitted through the bites of infected female phlebotomine sandflies, which feed on blood to produce eggs. Some 70 animal species, including humans, can be the source of Leishmania parasites. Leishmaniasis is endemic in more than 70 countries in the world, including regions of southern Europe, Northern Africa, the Middle East, Central Asia, China, South and Central America and has emerged also in dogs in the USA.

In humans, leishmaniasis typically presents as distinct visceral or mucocutaneous diseases, usually caused by distinct *Leishmania* species. In human visceral disease, the macrophages of the visceral reticuloendothelial system are parasitized by *L donovani* for which other humans are the reservoir, or *L infantum,* for which dogs are the main reservoir. In human cutaneous or mucosal disease, the macrophages of the skin or oro-nasal mucosa are parasitized by other *Leishmania* species (*L major, L tropica, L mexicana, L amazonensis, L braziliensis, L panamensis,* etc), although there are at least 2 examples where cutaneous disease is caused by "visceral" species: *L infantum* cutaneous disease and post-kala-azar dermal leishmaniasis.

Currently approved medicinal products for the treatment of human leishmaniasis include, in particular, antimonials, miltefosine and amphotericin B.

Antimony has been used as therapeutics in several centuries. The modern era of usage of antimony began in the early 20^{th} century. Use of the trivalent antimonial, tarteremetic was first reported for the treatment of CL in 1913, the efficacy was confirmed against VL in India in 1915, but later this drug was found to be highly toxic as well as very unstable in tropical climate. Later, the pentavalent antimony compound urea stibamine emerged as an effective chemotherapeutic agent against Indian kala-azar in 1920. The development of the less toxic pentavalent antimonials led to the synthesis of antimony gluconate (Solustibosan) in 1937 and sodium stibogluconate (Pentostam) in 1945. Nowadays the most commonly used organic compounds of antimony (Sb) are sodium antimony gluconate (SAG) and meglumine antimoniate (manufactured by Rhone-Poulence, Paris).

Miltefosine was developed as an orally administrable alternative to antimonials and was approved in 2014 for human visceral leishmaniasis at 2.5 mg/kg/day for 28 days.. Miltefosine has the following molecular structure:

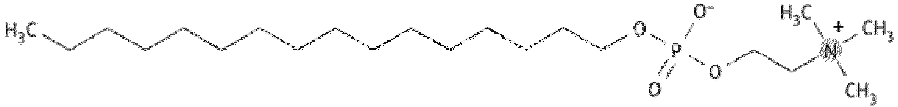

Amphotericin B may be used as a drug of last resort, but its use is limited by its need for IV administration and toxicity.

In relation to human leishmaniasis treatment various problems and concerns are generally seen, including (i) the need for parenteral administration of all drugs except miltefosine; (ii) declining efficacy of miltefosine in the region of largest prevalence, the Indian subcontinent, due to widespread use; and (iii) a lack of efficacy of amphotericin B and of miltefosine in Brazil, the region of next largest prevalence. For example, Carnielli et al. (Natural Resistance of Leishmania infantum to Miltefosine Contributes to the Low Efficacy in the Treatment of Visceral Leishmaniasis in Brazil. Am J Trop Med Hyg. 2019 Oct; 101(4): 789-794) describe that *L. infantum* amastigotes have been found resistant to miltefosine in isolates from VL patients in Brazil, who eventually failed treatment with miltefosine. According to Carnielli et al. their findings suggest natural resistance to this drug, as miltefosine had never been used in Brazil before the trial was carried out.

Although from the human point of view, canines are important as the animal reservoir for *L infantum,* the importance of dogs in veterinary medicine makes Canine Leishmaniasis a significant problem in its own right. Infection of dogs with *Leishmania infantum* is unlike human infection in that canine infection affects not just the visceral reticuloendothelial system, but also the skin, eyes, and kidney. In a longitudinal study in which initially *Leishmania*-naïve dogs in Europe were followed for 22 months, the month at which abnormalities were first seen was 6 months for lymph node enlargement, 6-22 months for exfoliative dermatitis, 12 months for the reversal of the normal albumin/globulin ratio, 12 months for anemia and increased creatinine/BUN, and 22 months for weight loss. The LeishVet group has provided a severity grading system for symptomatic animals that integrates clinical and laboratory abnormalities. Grade 1 (mild disease) signifies lymphadenopathy or papular dermatitis with creatinine <1.4 mg/dL; grade 2 (moderate disease) additionally have cutaneous lesions, anorexia/weight loss/fever, and anemia or hypergammaglobulinemia; grade 3 (severe disease) have in addition signs deriving from immune-complex lesions (including arthritis, uveitis and glomerulonephritis) and creatinine between 1.4 and 2.0 mg/dL; grade 4 (very severe disease) may have end stage renal disease with creatinine >2.9 mg/dL which implies that death of the dog is typically due to renal failure.

Current treatments of canine leishmaniasis typically involve use of one of the human leishmaniacidal agents meglumine antimoniate or miltefosine, often in combination with allopurinol, a drug compound not typically used for leishmaniasis treatment in humans. Allopurinol is a purine analog used mainly as a xanthine oxidase inhibitor to reduce serum urate concentration and is prescribed for the management of gout in humans. Allopurinol's antileishmanial activity was first described in 1974 and is attributed to the inhibition of the leishmanial enzyme hypoxanthine-guanine phosphoribosyl transferase (HGPRT). Allopurinol is an oral agent, but is leishmaniastatic not leishmaniacidal. Treatment of canine leishmaniasis usually includes a combined regimen with meglumine antimoniate or miltefosine administered for 4 weeks and allopurinol administered simultaneously and then continuously used for long-term therapy.

Although scarce, there are reports on drug resistance also in canine disease. Disease relapse in dogs with Canine leishmaniasis during allopurinol treatment has recently been described by Yasur-Landau et al. (Allopurinol Resistance in Leishmania infantum from Dogs with Disease Relapse. PLoS Negl Trop Dis. 2016 Jan; 10(1)), who reported allopurinol resistance of *L. infantum* isolated from relapsed animals. *Leishmania infantum* strains isolated in culture from relapsed dogs were significantly less susceptible to allopurinol in comparison to isolates from dogs before treatment and those from dogs under treatment with no clinical relapse. Resistance was consistent in three forms of the parasite strains tested including intracellular amastigotes, promastigotes and axenic amastigotes. These findings indicate that resistance to allopurinol may develop in dogs experiencing clinical disease relapse which may transmit resistant parasite to other dogs and also enhance the danger of transmission the parasite to humans.

There is an urgent need for new antileishmanial drugs that are effective and can be administered orally; at present, only miltefosine (milteforan) and alopurinol are available as oral therapy and their efficacy is declining for reasons of widespread use (Indian subcontinent) and inherent resistance (Brazil). The availability of new treatments, based on compounds not currently in use for treatment of leishmaniasis, not only provides a resort once drug resistance has developed, but also can be a tool in the development of strategies aimed at avoiding or substantially slowing down drug resistance development.

It is an object of the present invention to, amongst other things, provide such new treatments.

### Summary of the Invention

The present invention resides the surprising findings that the compound known as oleylphosphocholine (OlPC), having the molecular structure shown below, is highly effective, as an oral, daily treatment, in dogs and, remarkably, also in dogs suffering from leishmaniasis disease variants that do not display sufficient response to miltefosine treatment, as shown and explained in the examples.

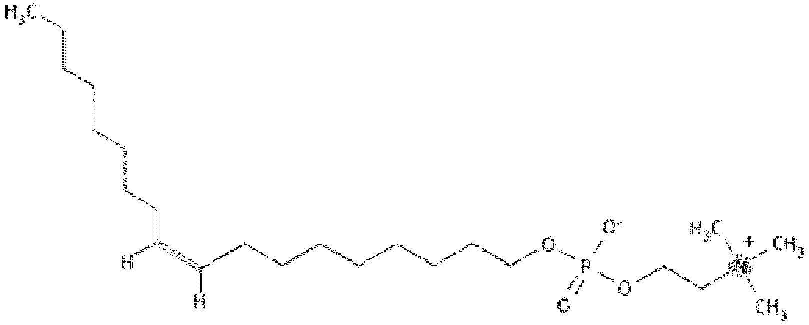

As can be seen, OlPC is an analog of miltefosine (hexadecyl phosphocholine) in which an oleyl group in OlPC replaces the hexadecyl group in miltefosine. Oleylphosphocholine has an unsaturated bond halfway the oleyl side chain. The present inventors now hypothesize that the increased chain rigidity, caused by the unsaturated bond, might cause different pharmacodynamic properties compared to miltefosine with its saturated side chain. This difference in pharmacodynamic properties could in turn explain why oleylphosphocholine is effective also in leishmaniasis disease variants that seem miltefosine treatment resistant, although the inventors are not bound by any theory in this regard. The present inventors believe that employing oleylphosphocholine treatment specifically for veterinary purposes might be effective as (part of) a strategy to reduce risks associated with the development of treatment resistance against the pharmacological treatments currently (also) in use for the human population.

The potential of OlPC as a treatment for leishmaniasis, and related (protozoan) diseases, has been the subject of research before. Animal studies conducted so far primarily were aimed at gathering safety data with a view to potential human use of OlPC.

Prior art document US 9,326,989 describes a (large) variety of potential uses of alkyl phosphocholines with cis double bonds, in particular OlPC, including uses for the treatment of protozoan diseases. The gist of US 9,326,989 appears to be that alkyl phosphocholines with cis double bonds, compared to saturated alkyl phosphocholines such as hexadecyl phosphocholine (miltefosine), can be used at *lower* daily dosages so as to improve the tolerability and safety profile. US 9,326,989 teaches that, in dogs, 0.3 mg of OlPC is the (daily) dose minimally required for activity, while 4.5 mg is the dose at which toxicity starts. On this basis, dosages of 0.1 to 0.9 mg/kg body weight per day are advocated. US 9,326,989 teaches that, because of the low OlPC dosages, the special formulations, in particular liposome formulations, typically used for other (saturated) alkyl phosphocholines to reduce safety/tolerability issues, are no longer needed. US 9,326,989 does not provide any efficacy and/or safety data for any of the suggested treatments/regimens.

In the present study, it has been demonstrated that OlPC, in a non-liposomal formulation, is efficacious at dosages of 4 mg/kg body weight per day (for 28 days), in dogs naturally infected with *L. infantum* (which were not treatable with miltefosine, at the standard/recommended dosages), while the treatment was well-tolerated. To the inventor's best knowledge this has not been demonstrated before.

A first aspect of the invention concerns a method for the therapeutic or prophylactic treatment of leishmaniasis in a non-human mammal the method comprising the oral administration of an effective amount of oleylphosphocholine to said mammal.

A further aspect of the invention concerns a method for the therapeutic or prophylactic treatment of a non-human mammal, wherein the non-human mammal suffers from leishmaniasis, is at risk of suffering from leishmaniasis, has been infected with *Leishmania* and/or is at risk of becoming infected with *Leishmania,* the method comprising the oral administration of an effective amount of oleylphosphocholine to said mammal.

A further aspect of the invention concerns a method of preventing a resurgence of *Leishmania* parasite load and/or maintaining a low *Leishmania* parasite load and/or suppressing *Leishmania* parasite load in a non-human mammal that suffers or has suffered from leishmaniasis disease, the method comprising the oral administration of an effective amount of oleylphosphocholine to said mammal.

A further aspect of the invention concerns a method of preventing leishmaniasis disease relapse in a non-human mammal, the method comprising the oral administration of an effective amount of oleylphosphocholine to said mammal.

A further aspect of the invention concerns a veterinary (pharmaceutical) composition comprising oleylphosphocholine for use in a therapeutic or prophylactic method of treating leishmaniasis in a non-human mammal, wherein the non-human mammal suffers from leishmaniasis, is at risk of suffering from leishmaniasis, has been infected with *Leishmania* and/or is at risk of becoming infected with *Leishmania,* the method comprising the oral administration of an effective amount of oleylphosphocholine to said mammal.

A further aspect of the invention concerns a veterinary (pharmaceutical) composition comprising oleylphosphocholine for use in a therapeutic or prophylactic method of treating a non-human mammal, the method comprising the oral administration of an effective amount of oleylphosphocholine to said mammal.

A further aspect of the invention concerns a veterinary (pharmaceutical) composition comprising oleylphosphocholine for use in a method of preventing a resurgence of *Leishmania* parasite load and/or maintaining a low *Leishmania* parasite load and/or suppressing *Leishmania* parasite load in a non-human mammal that suffers or has suffered from leishmaniasis disease, the method comprising the oral administration of an effective amount of oleylphosphocholine to said mammal.

A further aspect of the invention concerns a veterinary (pharmaceutical) composition comprising oleylphosphocholine for use in a method of preventing leishmaniasis disease relapse in a non-human mammal, the method comprising the oral administration of an effective amount of oleylphosphocholine to said mammal.

A further aspect of the invention concerns the use of oleylphosphocholine in the manufacture of a veterinary (pharmaceutical) composition for use in a method for the therapeutic or prophylactic treatment of leishmaniasis in a non-human mammal, the method comprising the oral administration of an effective amount of oleylphosphocholine to said mammal.

A further aspect of the invention concerns the use of oleylphosphocholine in the manufacture of a veterinary (pharmaceutical) composition for use in a method for the therapeutic or prophylactic treatment of a non-human mammal, wherein the non-human mammal suffers from leishmaniasis, is at risk of suffering from leishmaniasis, has been infected with *Leishmania* and/or is at risk of becoming infected with *Leishmania,* the method comprising the oral administration of an effective amount of oleylphosphocholine to said mammal.

A further aspect of the invention concerns the use of oleylphosphocholine in the manufacture of a veterinary (pharmaceutical) composition for use in a method of preventing a resurgence of *Leishmania* parasite load and/or maintaining a low *Leishmania* parasite load and/or suppressing *Leishmania* parasite load in a non-human mammal that suffers or has suffered from leishmaniasis disease, the method comprising the oral administration of an effective amount of oleylphosphocholine to said mammal.

A further aspect of the invention concerns the use of oleylphosphocholine in the manufacture of a veterinary (pharmaceutical) composition for use in a method of preventing leishmaniasis disease relapse in a non-human mammal, the method comprising the oral administration of an effective amount of oleylphosphocholine to said mammal.

In particularly preferred embodiments of the invention the non-human mammal is a dog, as will be understood by those skilled in the art, based on the results presented herein. Other particularly preferred embodiments of the invention relate to the treatment of disease variants wherein miltefosine treatment has limited or no effect, such as disease caused by infection with miltefosine resistant *Leishmania* parasites. It will also be understood, based on the results presented herein, that particularly preferred embodiments relate to treatments intended to achieve and/or resulting in substantially or complete suppression of *Leishmania* parasite load for prolonged periods of time, such as at least 3 months.

A further aspect of the invention concerns a veterinary (pharmaceutical) dosage form that is adapted, suitable and/or intended for use in the methods defined herein. In preferred embodiments, such dosage forms comprise oleylphosphocholine in the form of a granulate that:
i) comprises a mixture of oleyl phosphocholine, an anti-oxidant, a filler and, optionally, and/or one or more further granulation excipients and
ii) is obtainable by a melt-agglomeration process, preferably a hot-melt extrusion process, or a wet granulation process, preferably a high-shear wet granulation process. In further preferred embodiment the dosage form is specifically adapted for use in veterinary indications.

These aspects, as well as their preferred embodiments are further described and illustrated in the following detailed description and examples.

### Detailed description of the Invention

### Products and compositions

As indicated above, in an aspect, veterinary (pharmaceutical) compositions comprising Oleyl Phosphocholine are provided that, typically, are adapted, suitable and/or intended for use in the methods defined herein. Oleyl phosphocholine is the common name for the compound having molecular formula C₂₃H₄₈NO₄P and the molecular structure shown here below. It has CAS no. 76622-80-5.

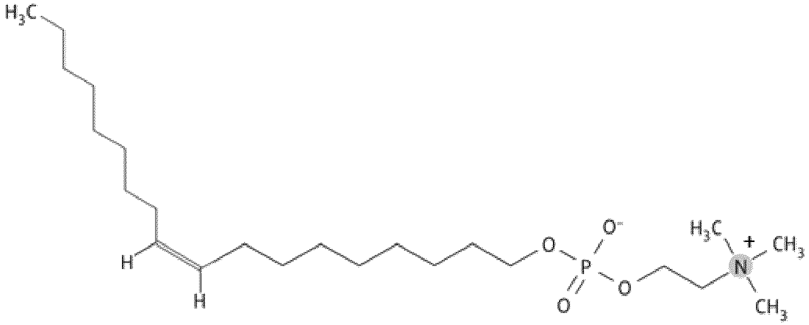

In the context of the present invention, the term "veterinary (pharmaceutical) composition" refers to a composition comprising OlPC in combination with one or more additional, non-toxic or physiologically inert, ingredients, in particular one or more pharmaceutically acceptable carriers and/or excipients, which composition typically is in a form suitable for administration to an animal, through any route of administration, and which composition is physiologically tolerated when administered to an animal subject (at dosages that are in accordance with the present teachings). The use of the term "pharmaceutical" (in brackets) serves to specify that the compositions, typically, are intended for use in veterinary medicine, as will be understood by those skilled in the art, based on the present teachings..

In preferred embodiments of the invention, the veterinary (pharmaceutical) composition comprises OlPC in non-liposomal form. More in particular, the veterinary (pharmaceutical) composition of the invention preferably does not comprise, besides OlPC, any lipids. In certain preferred embodiments, the relative amount of lipidic components, excluding OlPC, in the veterinary (pharmaceutical) composition of the invention is below 10 % (w/w) based on the total weight of the composition, below 5 % (w/w), below 4 % (w/w), below 3 % (w/w), below 2 % (w/w), below 1 % (w/w), below 0.5 % (w/w) or below 0.1 % (w/w). In certain preferred embodiments the veterinary (pharmaceutical) composition of the invention does not contain, besides OlPC, any phospholipids, such as phophatidylcholines; any glycerophospholipids; any phosphatidylpolyethylene glycols; and/or cholesterol. In other preferred embodiments, the veterinary (pharmaceutical) composition does not comprise OlPC in (micro)encapsulated form, more preferably, it does not comprise OlPC (micro)encapsulated in one or more lipidic components; OlPC (micro)encapsulated within a lipid bilayer; OlPC contained within a lipidic vesicle; and/or OlPC (micro)encapsulated within a lipidic matrix.

In preferred embodiments of the invention, the veterinary (pharmaceutical) composition comprises OlPC in the form of a granulate. In the context of the present invention, the term "granulate", in line with what is generally understood, refers to aggregates of particles, sometimes called granules (Remington's Pharmaceutical Sciences 18th ed. 1990, page 1641).

Suitable granulates can typically comprise OlPC at a (relative) amount of at least 5 wt.%, based on the total weight of the granulate, preferably at least 10 wt.%, at least 15 wt.%, at least 17.5 wt.%, at least 20 wt.%, at least 22.5 wt.% or at least 25 wt.%, and/or at a relative amount of less than 60 wt.%, less than 50 wt.%, less than 45 wt.%, less than 40 wt.%, or less than 35 wt.%, such as a (relative) amount of between 10 and 50 wt.%, preferably between 25 and 35 wt.%. In particularly preferred embodiments of the invention, veterinary (pharmaceutical) compositions are provided, wherein the concentration of oleyl phosphocholine in the OlPC containing granulate is between 10 and 50 wt.%, preferably between 25 and 35 wt.%.

In preferred embodiments of the invention, the veterinary (pharmaceutical) composition comprises a granulate, said granulate comprising a mixture of olelyl phosphocholine, an anti-oxidant, a filler and, optionally, one or more further granulation excipients.

In an embodiment, the antioxidant is selected from the group consisting of alpha tocopherol, alpha tocopherol acetate, Vitamin E, Vitamin E TPGS, diethylhexyl syringylidene malonate, diisopropyl vanillidene malonate, tetrahydrocurcumenoids, tocopherol, carotenoids, anthocyanidins, hydroquinone monomethyl ether, ascorbic acid, butylated hydroxyanisole (BHA), butylated hydroxytoluene (BHT), tert-butylhydroquinone (TBHQ), propyl gallate and ethoxyquin (EMQ) and mixtures thereof. In a particularly preferred embodiment of the invention the antioxidant is selected from the group consisting of alpha tocopherol, alpha tocopherol acetate, Vitamin E and Vitamin E TPGS. In a particularly preferred embodiment of the invention the antioxidant is Vitamin E or alpha tocopherol.

In an embodiment, the filler is selected from the group consisting of calcium carbonate, calcium phosphate (dibasic), calcium phosphate (tribasic), calcium sulphate, cellulose, microcrystalline cellulose, microcrystalline silicified cellulose, powdered cellulose, dextrates, dextrose, fructose, lactitol, lactose monohydrate, magnesium carbonate, maltitol, maltodextrin, maltose, mannitol, sodium chloride, sorbitol, starch, pregelatinized starch, sucrose, compressible sugar, sugar spheres, talc, xylitol, silicium dioxide, such as colloidal silicone dioxide (colloidal silica), silica gel, mesoporous silica or nanoporous silica; and mixtures thereof. In a particularly preferred embodiment of the invention, the filler is selected from the group consisting of cellulose, microcrystalline cellulose, microcrystalline silicified cellulose, powdered cellulose, silicium dioxide, such as colloidal silicone dioxide (colloidal silica), silica gel, mesoporous silica or nanoporous silica; and mixtures thereof.

In preferred embodiments of the invention, the veterinary (pharmaceutical) composition comprises a granulate, said granulate comprising, besides OlPC, the anti-oxidant and the filler, one, two or all of the following granulation excipients: a spacer, disintegrants, binders and lubricants.

The spacer that may be present as a granulation excipient is preferably hydrophilic polymers, more preferably selected from the group consisting of polyethylene glycol, polyethylene glycol esters, polypropylene glycol, polypropylene glycol esters, polyvinyl pyrrolidone (PVP), poly-(2-oxazoline)s (POX) and polyacrylic acid (PAA) and hydroxypropylmethylcellulose. In one particularly preferred embodiment of the invention, the spacer is a hydrophilic polymer selected from the group consisting of polyethylene glycol 4000 (PEG 4000, macrogol 4000) and polyethylene glycol 6000 (PEG 6000, macrogol 6000), polyvinyl pyrrolidone (PVP), poly-(2-oxazoline)s (POX) and polyacrylic acid (PAA). More preferably, the granulation spacer in a process for agglomeration is polyethylene glycol 4000 or polyethylene glycol 6000, more preferably polyethylene glycol 6000. **In** certain embodiments of the invention, products comprising PEG esters in combination with other components may be used as the spacer, such as the hydrophilic grades of Gelucire, e.g. Gelucire 50/13, 44/14, 48/,16, 55/18, 35/10 and 48/09, which are based on mixtures of mono, di and triglycerides with PEG esters of fatty acids.

The disintegrant that may be present as granulation excipient is preferably selected from the group consisting of starch, microcrystalline cellulose, alginic acid, methyl cellulose, sodium starch glycolate, croscarmellose sodium, crospovidone, calcium silicate and mixtures thereof.

The binder that may be present as granulation excipient is preferably selected from the group consisting of acacia mucilage, alginic acid, carbomer, carboxymethylcellulose calcium, carboxymethycellulose sodium, microcrystalline cellulose, powdered cellulose, ethyl cellulose, gelatine, liquid glucose, guar gum, hydroxyethyl cellulose, hydroxypropyl cellulose, low substituted hydroxypropyl cellulose, hydroxypropylmethyl cellulose (hypromellose), magnesium aluminum silicate, maltodextrin, methylcellulose, polydextrose, polyethylene oxide, povidone, copovidone, sodium alginate, starch paste, pregelatinized starch, sucrose (syrup), silicium dioxide and mixtures thereof. Preferably, the silicium dioxide is colloidal silicone dioxide (colloidal silica), silica gel, mesoporous silica, nanoporous silica.

The lubricant that may be present as granulation excipient is preferably selected from the group consisting of calcium stearate, fumaric acid, glyceryl behenate, glyceryl palmitostearate, hydrogenated vegetable oil, magnesium lauryl sulphate, magnesium stearate, sodium lauryl sulphate, sodium stearyl fumarate, starch, stearic acid, talc, zinc stearate and mixtures thereof. **In** a specific embodiment is provided a process for agglomeration according to the invention wherein the granulation lubricant is selected from said group. In a specific embodiment is provided a process for granulation according to the invention wherein the granulation lubricant is selected from said group. In another specific embodiment is provided a process for preparing a tablet dosage formulation according to the invention wherein the granulation lubricant and the compression lubricant are independently selected from said group.

In an embodiment, the veterinary (pharmaceutical) composition comprises a granulate, wherein:
- the granulate contains OlPC at a relative amount of between 10 and 50 wt.%, based on the total granulate weight, preferably between 25 and 35 wt.%;
- the granulate contains antioxidant at a relative amount of between 0.05 and 5 wt.%, based on the total granulate weight, preferably between 0.1 and 1 wt.%, preferably wherein said antioxidant is selected from the group consisting of alpha tocopherol, vitamin E, alpha tocopherol acetate, ascorbic acid, butylated hydroxyanisole (BHA), butylated hydroxytoluene (BHT), tert-butylhydroquinone (TBHQ), propyl gallate and ethoxyquin and mixtures thereof, most preferably wherein said antioxidant is alpha tocopherol or Vitamin E;
- the granulate contains the binder, if present, at a relative amount of between 10 and 50 wt.%, based on the total granulate weight, preferably between 25 and 35 wt.%, preferably wherein said binder is selected from the group consisting of acacia mucilage, alginic acid, carbomer, carboxymethylcellulose calcium, carboxymethycellulose sodium, microcrystalline cellulose, powdered cellulose, ethyl cellulose, gelatine, liquid glucose, guar gum, hydroxyethyl cellulose, hydroxypropyl cellulose, low substituted hydroxypropyl cellulose, hydroxypropylmethyl cellulose, magnesium aluminum silicate, maltodextrin, methylcellulose, polydextrose, polyethylene oxide, povidone, copovidone, sodium alginate, starch paste, pregelatinized starch, sucrose (syrup) and mixtures thereof, most preferably wherein said binder is microcrystalline cellulose;
- the granulate contains the spacer, if present, at a relative amount of between 10 and 50 wt.%, based on the total granulate weight, preferably between 25 and 35 wt.%, preferably wherein said spacer is selected from the group consisting of polyethylene glycol 4000, polyethylene glycol 6000, polyvinyl pyrrolidone, poly-(2-oxazoline)s and polyacrylic acid and mixtures thereof, most preferably wherein said spacer is polyethylene glycol 6000;
- the granulate contains the disintegrant, if present, at a relative amount of between 1 and 20 wt.%, based on the total granulate weight, preferably between 1 and 10 wt.%, preferably wherein said disintegrant is selected from the group consisting of starch, microcrystalline cellulose, alginic acid, methyl cellulose, sodium starch glycolate, croscarmellose sodium, crospovidone, calcium silicate and mixtures thereof, most preferably wherein said disintegrant is croscarmellose sodium; and
- the granulate contains the lubricant, if present, at a relative amount of between 1 and 20 wt.%, based on the total granulate weight, preferably between 1 and 10 wt.%, preferably wherein said lubricant is selected from the group consisting of calcium stearate, fumaric acid, glyceryl behenate, glyceryl palmitostearate, hydrogenated vegetable oil, magnesium lauryl sulphate, magnesium stearate, sodium lauryl sulphate, sodium stearyl fumarate, starch, stearic acid, talc, zinc stearate and mixtures thereof, most preferably wherein said lubricant is selected from the group consisting of magnesium stearate and mixtures thereof.

Methods of producing granulates that are considered particularly suitable for use in the present pharmaceutical and veterinary compositions are disclosed in prior art documents WO2020208230 and WO2021204409. These documents disclose melt-granulation and wet granulation processes. Melt-agglomeration, carried out at a temperature above the melting temperature of OlPC, so that OlPC is present (primarily) in a liquid state during the process, results in the agglomeration/binding of primary particles of the granulation excipient and of the OlPC. In certain preferred embodiments, the melt-agglomeration step comprises hot-melt extrusion. Alternatively, different (wet) agglomeration techniques can be employed to produce the granulate, such as extrusion granulation, high-shear granulation, low-shear granulation, or fluidized bed granulation techniques.

Hence, in embodiments of the invention, the veterinary (pharmaceutical) composition comprises a granulate, wherein the granulate is a granulate obtainable by a melt-agglomeration process, preferably a hot-melt extrusion process, or a wet granulation process, preferably a high-shear wet granulation process. In embodiments of the invention, the veterinary (pharmaceutical) composition comprises a granulate, wherein said granulate:
i) comprises a mixture of olelyl phosphocholine, an anti-oxidant, a filler and, optionally, and/or one or more further granulation excipients and
ii) is obtainable by a melt-agglomeration process, preferably a hot-melt extrusion process, or a wet granulation process, preferably a high-shear wet granulation process; and wherein the dosage form is adapted for providing one or more metered amounts of the granulate comprising the correct dose of oleylphosphocholine.

In preferred embodiments of the invention, the veterinary (pharmaceutical) composition comprises a granulate, wherein the granulate is a granulate obtainable by a process comprising the consecutive steps of:
a) preparing a mixture of oleyl phosphocholine, an anti-oxidant, a filler and, optionally, a granulation liquid and/or one or more further granulation excipients;
b) processing the mixture prepared in step a) into a dry granulate; and
c) milling the dry granulate produced in step b).

In some embodiments, the mixture produced in step a) of the process, is heated typically to a temperature above the melting point of OlPC and said heated mixture is subjected to mechanical shear force. In preferred embodiments, the melt-agglomeration step, such as a hot melt extrusion step, is carried out at a temperature of at least 50 °C, e.g. at a temperature of at least 60 °C, at least 65 °C, at least 70 °C or at least 75 °C. Preferably the melt-agglomeration step, is carried out at a temperature below 120 °C, e.g. at a temperature below 100 °C, preferably below 90 °C. In preferred embodiments, no solvent is added during any one of steps a), b) and c) of the process. In a preferred embodiment of the invention, step b) comprises a hot melt extrusion step, using (e.g.) a twin screw extruder, operated such that the temperature of the granulation mixture is maintained between 40°C and 60°C.

In some other embodiments, step a) comprises the addition, to the mixture, of a granulation liquid. The granulation liquid may be any liquid capable of dissolving the OlPC, such as water or certain organic solvents, to the extent suitable for use in pharmaceutical manufacturing, i.e. with a view to safety/toxicity. Preferably a granulation liquid selected from the group consisting of from isopropyl alcohol (IPA), acetone, ethanol, dichloromethane, chloroform, water and mixtures thereof. In a particularly preferred embodiment of the invention, the process comprises the step of dissolving OlPC in the granulation liquid. In certain preferred embodiments, the antioxidant is vitamin E or alpha tocopherol and the anti-oxidant is added to the granulation liquid as well. Hence, in embodiments, step a) comprises: a1) providing a dry powder (blend) of the filler and optional further granulation excipients; a2) preparing a solution of the OlPC and anti-oxidant in the granulation liquid; and adding to the dry powder (blend) of step a1), the solution prepared in step a2). In these processes, wherein a granulation liquid is used, step b) typically comprises subjecting the mixture to a mechanical operation, such as extrusion granulation, high-shear granulation, low-shear granulation, or fluidized bed granulation processes. In particularly preferred embodiments, (high) shear-mixing or extrusion is carried out, e.g. by means of a high shear mixer or a twin-screw extruder, and subsequently drying the obtained granulate. Thus, in accordance with this embodiment, step b) comprises subjecting the mixture to high shear granulation or extrusion granulation, followed by drying of the obtained granulate.

OlPC and the one or more granulation excipients can be combined and mixed in any order. In certain embodiments premixes of various combinations of the respective components may be made, which pre-mixes are then combined and blended. In preferred embodiments of the invention, the blending step comprises intimate blending or mixing of the OlPC and granulation excipients to produce a homogeneous blend or mixture, employing techniques such as roller mixing, drum mixing, shear mixing, dry blending, chopping, milling, etc.

In accordance with the various aspects of the invention, the composition is preferably provided in unit dosage form. The term "unit dosage form" refers to a physically discrete unit suitable as a unitary dosage for human subjects, each unit containing a predetermined quantity of active material calculated to produce the desired therapeutic effect in association with any suitable pharmaceutical carrier(s) and/or excipient(s). Exemplary, non-limiting unit dosage forms include a tablet, caplet, capsule (e.g., a hard capsule or a soft capsule), lozenge, film, strip, gelcap as well as any metered volume of a powder, granulate, solution, suspension, syrup or elixir or the like, which may be contained, for instance in a vial, syringe, applicator device, sachet, spray, micropump etc. In accordance with particularly preferred embodiments of the invention, the unit dosage form, is a unit dosage form that is suitable for oral administration. Most preferably, it is a solid unit dosage form, such as a tablet for oral ingestion or a powder or granulate that can be added directly to the feed of the non-human mammal and/or that can be fed to the non-human mammal after (re)dispersion in (tap)water.

In accordance with various aspects of the invention, the composition is provided in a unit dosage form comprising oleyl phosphocholine in a dose of at least 2 mg, preferably at least 4 mg, at least 4.5 mg, at least 4.6 mg, at least 5 mg, at least 8 mg, at least 16 mg, at least 20 mg, at least 25 mg, at least 30 mg, at least 35 mg, at least 40 mg, at least 45 mg, or at least 50 mg. In accordance with the various aspects of the invention, the composition is typically provided in a unit dosage form comprising oleyl phosphocholine in a dose of 500 mg or less, more preferably 400 mg or less, 300 mg or less, 250 mg or less, 200 mg or less, 175 mg or less, 150 mg or less, 125 mg or less, 110 mg or less, 100 mg or less, 95 mg or less, 90 mg or less, 84 mg or less or 80 mg or less. In accordance with the various aspects of the invention, the composition is preferably provided in a unit dosage form comprising oleyl phosphocholine in a dose within the range of 2-500 mg, 25-250 mg, or 50-100 mg.

In other preferred embodiments of the invention, the composition is provided as a finished formulation or dosage form that is adapted for division into two or more fractions, providing one or more metered amounts of the granulate comprising, approximately, a predetermined dose of oleylphosphocholine. As will be understood by those skilled in the art, based on the present teachings, in the case of veterinary applications, it can be highly advantageous to provide a finished formulation or form that allows a veterinarian or a pet owner, in an easy way, to obtain an adjusted dose appropriate for the animal's size and weight. For instance, for these purposes, the composition can be provided in the form of a tablet, comprising OlPC, preferably in the form of a granulate as defined herein before, at quantities adequate for single dosing of (e.g.) large dogs, said tablet being provided with one or more score lines, along which the tablet can be split to yield two (or more) parts that contain a predetermined dose (by approximation), suitable for smaller dogs. Alternatively, a finished product can be provided that has the form of a granulate, preferably a granulate as defined herein before, packaged, e.g. in sachets, whereby each sachet contains OlPC at quantities adequate for single dosing of (e.g.) large dogs, said finished product further being provided with means that allow the user to take an adequately metered amount of the granulate that contains a predetermined dose (by approximation), suitable for smaller dogs.

### Therapeutic Indications & End-points

As explained herein before, the methods of the invention, comprising the administration of OlPC to a non-human mammal, typically in the form of a pharmaceutical composition as defined herein, can be therapeutic as well as prophylactic in nature.

In certain embodiments of the invention, methods are provided for the treatment and/or prevention of leishmaniasis in a non-human mammal in need thereof.

In certain embodiments of the invention, methods are provided for curative or prophylactic treatment of non-human mammals suffering from or at risk of attracting leishmaniasis and/or non-human mammals infected with and/or at risk of becoming infected with *Leishmania.*

The terms "treat", "treating" or "treatment" as used herein, when appearing in conjunction with a specific disorder or symptom (for example: "method of treating leishmaniasis") refers to a method of curing, alleviating or abrogating said disorder and/or accompanying symptoms.

The terms "prevent", "preventing" or "prevention", as used herein, refer to a method of barring a subject from acquiring a disorder and/or its attendant symptoms. In certain embodiments, the terms "prevent", "preventing" or "prevention" refer to a method of reducing the risk of acquiring a disorder and/or accompanying symptoms. Embodiments of the present invention wherein methods are aimed at preventing reactivation of existing disease, also referred to herein as dormant or latent disease, may also be referred to as relating to 'prevention' and/or as being 'prophylactic'.

The terms "treat", "treating" or "treatment", when used in relation to a patient or subject (for example: "method of treating a subject"), typically refers to the act of administering a therapeutic compound to said patient or subject for whatever therapeutic and/or prophylactic purpose, as will immediately be apparent from the definitions and context.

As used herein the term 'leishmaniasis' denotes a vector-borne condition, characterized by a wide variety of clinical manifestations, caused by intracellular protozoa of the genus *Leishmania,* which are transmitted by the bite of infected female phlebotomine sandflies. There are over 20 *Leishmania* species that cause leishmaniasis in mammals (including humans), including *L. donovani, L. infantum,* and *L. chagasi, L. mexicana, L. amazonensis, and L. venezuelensis, L. tropica, L. major, L. aethiopica, L. (V.) braziliensis, L. (V.) guyanensis, L. (V.) panamensis,* and *L. (V.) peruviana.* In preferred embodiments of the invention, the leishmaniasis to be treated or prevented, is leishmaniasis caused by any of the aforementioned parasites. In a particularly preferred embodiments of the invention, the leishmaniasis to be treated or prevented is leishmaniasis caused by *L. infantum.* In other preferred embodiments of the invention, the leishmaniasis to be treated or prevented is leishmaniasis caused by a miltefosine (treatment) resistant *Leishmania* species or strain. In certain particularly preferred embodiments of the invention, the leishmaniasis to be treated or prevented is leishmaniasis caused by a miltefosine (treatment) resistant *L. infantum* strain. As used herein, the terms 'miltefosine resistant' and 'miltefosine treatment resistant' are considered to be synonymous. Whether a *Leishmania* strain is miltefosine (treatment) resistant can be established by a person skilled in the art by routine investigations/experimentation. In one embodiment, a *Leishmania* strain is considered miltefosine (treatment) resistant in case the miltefosine IC₅₀ value in an appropriate *in vitro* assay is above a certain threshold level, for instance above 4 µM, such as above 5 µM, above 6 µM, above 7 µM, above 8 µM, above 9 µM or above 10 µM. A suitable *in vitro* assay is described, for instance, in Carnielli et al. (Natural Resistance of Leishmania infantum to Miltefosine Contributes to the Low Efficacy in the Treatment of Visceral Leishmaniasis in Brazil. Am J Trop Med Hyg. 2019 Oct; 101(4): 789-794), the respective contents of which are incorporated herein by reference. Hence, in a preferred embodiment a *Leishmania* strain is considered miltefosine (treatment) resistant in case the miltefosine IC₅₀ value is above the threshold levels indicated above, as determined in an *in vitro* assay comprising the steps of: (i) infecting adherent macrophages from peritoneal fluid of Swiss mice with late-log phase promastigotes at a ratio of seven parasites to one macrophage, using the 16-well Lab-Tek tissue culture slides (Nunc, NY); (ii) incubating for 24 hours supplemented with 10% Hi-FCS in 5% CO2 at 37°C; (iii) removing free promastigotes; (iv) exposing the culture to different concentrations of miltefosine (0, 0.55, 1.67, 5, and 15 µM) in triplicate; (v) incubating for 72 hours; (vi) staining the slides with Diff-Quick solutions; (vii) counting 100 cells in each well to determine the percentage of infected macrophages; (viii) determining drug activity from the percentage of infected cells in drug-treated cultures relative to nontreated cultures, wherein the half-maximal inhibitory concentrations (IC₅₀) of miltefosine for amastigotes is calculated from nonlinear regression analysis, using GraphPad Prism v.7.0a software (GraphPad Software, San Diego, CA). The IC₅₀ value is preferably expressed as the mean of a number of independent experiments, such as at least 2, at least 3, at least 4 or at least 5 experiments. In particularly preferred embodiments of the invention, the leishmaniasis to be treated or prevented is leishmaniasis caused by strains of *L. infantum* indigenous to areas located in the Americas, including North-America and South-America, more preferably strains of *L. infantum* indigenous to areas located in Mexico, Argentina, Bolivia, Brazil, Chile, Colombia, Ecuador, Guyana, Paraguay, Peru, Suriname, Uruguay, or Venezuela, most preferably strains of *L. infantum* indigenous to areas located in Brazil.

There are 3 main forms of leishmaniases: visceral, which is the most severe form and almost always fatal without treatment; cutaneous, which is the most common form; and mucocutaneous.

In one preferred embodiment, the leishmaniasis is cutaneous leishmaniasis. Cutaneous leishmaniasis causes dermal lesions, mainly ulcers, on exposed parts of the body. These can leave life-long scars and cause disability. Clinical signs of the cutaneous form most commonly include dermal lesions, ocular abnormalities, epistaxis and hyperkeratosis. Many dogs will lose the pigment or dark coloring of these tissues as the disease progresses. Nodules or hard lumps may form in the skin and the coat often appears dull and brittle with areas of hair loss. The nails may grow long and curve abnormally.

**In** one preferred embodiment, the leishmaniasis is visceral leishmaniasis. Visceral leishmaniasis is characterized by irregular bouts of fever, weight loss, enlargement of the spleen and liver, and anemia. Visceral form is fatal if left untreated in over 95% of cases. Clinical signs of the visceral form include fever, anorexia (lack of appetite), weakness, decreased stamina, severe weight loss, diarrhea, vomiting, increased drinking and urination, and bleeding from the nose

**In** one preferred embodiment, the leishmaniasis is mucocutaneous leishmaniasis. Mucocutaneous leishmaniasis is usually a complication of cutaneous leishmaniasis and leads to partial or total destruction of mucous membranes of the nose, mouth and throat

**In** particularly preferred embodiments, the present invention concerns the treatment of dogs. Hence, in accordance with the invention, the leishmaniasis preferably is canine leishmaniasis, more preferably canine visceral leishmaniasis or canine cutaneous leishmaniasis. Furthermore, in preferred embodiments, the canine leishmaniasis is leishmaniasis caused by and/or associated with infection by *Leishmania infantum,* more preferably a by a miltefosine (treatment) resistant strain of *L. infantum.*

In one preferred embodiment, curative or therapeutic methods are provided for treating one or more symptoms associated with leishmaniasis in a non-human mammal in need thereof. In particularly preferred embodiments of the invention, the methods result in and/or are aimed at and/or are intended for one or more of the following (as compared to baseline or start of treatment): a reduction in or normalization of overall clinical Score; a reduction in and/or normalization of lymph node size; a reduction in and/or normalization of spleen size; improvement in and/or normalization of mucosal color; improvement in and/or normalization of nutritional status; a reduction in and/or reversal/disappearance of periocular dermatitis; a reduction in and/or reversal/disappearance of conjunctivitis; a reduction in or reversal/disappearance of uveitis; a reduction in and/or reversal/disappearance of vasculitis; a reduction in and/or reversal/disappearance of crust formation on the animal's ear tip(s); a reduction in and/or reversal/disappearance of onychogryphosis; a reduction in and/or reversal/disappearance of ulcers; a reduction in and/or reversal/disappearance of depigmentation; a reduction in and/or reversal/disappearance of hyperkeratosis; a reduction in and/or reversal of alopecia; and a reduction in and/or reversal/disappearance of furfuraceous dermatitis. As will be understood by those skilled in the the term 'clinical score', as used herein can refer to different scoring systems that have been proposed/developed in this field. In one embodiment, the Leish Vet scoring system is used, and the methods as defined herein result in and/or are aimed at and/or are intended for a reduction in and/or normalization of LeishVet clinical grading score, e.g. a reduction to 1 or lower, such as a reduction to 0. In another embodiment, a scoring system is used as defined in the examples below, wherein 23 parameters are graded 0 (normal), 1 (somewhat abnormal), or 2 (more abnormal) according to Table 1 of example 1, after which the grades for all clinical parameters are summed to give an overall clinical score for that animal, and the methods as defined herein result in and/or are aimed at and/or are intended for a reduction in said score and/or normalization of said score, e.g. a reduction to 1 or lower, such as a reduction to 0. This scoring system has also been described by dos Santos Noguiera et al. (Use of miltefosine to treat canine visceral leishmaniasis caused by Leishmania infantum in Brazil. Parasit Vectors. 2019 Feb 8;12(1):79), the respective contents of which are incorporate herein by reference.

In other preferred embodiments, prophylactic methods are provided for preventing one or more symptoms associated with leishmaniasis in a non-human mammal in need thereof. In particularly preferred embodiments of the invention, the methods result in and/or are aimed at and/or are intended for one or more of the following: preventing an increase in overall clinical Score and/or maintaining normal clinical score; preventing enlargement of lymph nodes and/or maintaining normal lymph node size; preventing spleen enlargement and/or maintaining normal spleen size; preventing mucosal discoloration (or hypocoloration) and/or maintaining normal mucosal color, preventing a worsening of nutritional status and/or maintaining normal nutritional status, preventing periocular dermatitis; preventing conjunctivitis; preventing uveitis; preventing vasculitis; preventing crust formation on the animal's ear tip(s); preventing onychogryphosis; preventing ulcers; preventing depigmentation and/or maintaining normal pigmentation; preventing hyperkeratosis; preventing alopecia; and preventing furfuraceous dermatitis. In one embodiment, the LeishVet scoring system is used, and the methods as defined herein result in and/or are aimed at and/or are intended for preventing an increase in LeishVet clinical grading score and/or maintaining a LeishVet clinical grading score of 1 or lower, preferably a score of 0. In another embodiment, a scoring system is used as defined in the examples below and as described in the article of Dos Santos Nogueira et al. as referenced above, and the methods as defined herein result in and/or are aimed at and/or are intended for preventing an increase in said score and/or maintaining a score of 1 or lower, preferably a score of 0.

As is known by those skilled in the art, survival of the parasite after apparent clinical cure is a problem in leishmaniasis treatment/management. Persistence of parasites, after (apparent) clinical cure of leishmaniasis, in the lymph nodes can result in clinical disease relapses or reactivation of disease, typically months after the onset of primary disease.

Hence, in preferred embodiments, the methods provided are effective in and/or are aimed at and/or are intended for preventing of persistence of parasites in the non-human mammal. In other preferred embodiments of the invention the methods provided are effective in and/or are aimed at and/or are intended for the substantially complete or complete eradication of *Leishmania* parasites in the non-human mammal.

Furthermore, in preferred embodiments, the methods provided are effective in and/or are aimed at and/or are intended for preventing, delaying or reducing the severity of leishmaniasis disease relapse and/or preventing, delaying or reducing the severity of dormant/latent leishmaniasis reactivation in a non-human mammal.

Furthermore, in preferred embodiments, the methods provided are effective in and/or are aimed at and/or are intended for substantially or completely suppressing leishmaniasis disease symptoms, such as one or more of the symptoms defined herein before, typically for a period of at least 3 months, preferably at least 4 months, more preferably at least 5 months.

Furthermore, in preferred embodiments, the methods provided are effective in and/or are aimed at and/or are intended for establishing/maintaining a leishmaniasis disease free interval or period of at least 3 months, preferably at least 4 months, more preferably at least 5 months.

Furthermore, in preferred embodiments, the methods provided are effective in and/or are aimed at and/or are intended for preventing a resurgence of *Leishmania* parasite load and/or maintaining a low Leishmania parasite load and/or suppressing *Leishmania* parasite load in a non-human mammal that suffers or has suffered from leishmaniasis disease, typically for a period of at least 3 months, preferably at least 4 months, more preferably at least 5 months.

In the context of the present invention, parasite load can typically refer to skin parasite load as well as to splenic parasite load. It is within the normal capabilities of the person skilled in the art to determine skin and/or splenic parasite loads. In particularly preferred embodiments of the invention, the term 'low parasite load' and related terms refer to a splenic parasite load, expressed as the number of parasites per ml of splenic aspirate, of below 50,000, preferably below 25,000, below 10,000, below 5,000, below 2,500, below 1,000, below 500, below 250 or below 100. Hence, in preferred embodiments, the methods provided are effective in and/or are aimed at and/or are intended for preventing a resurgence of Leishmania parasite load to above any of the aforementioned levels, and/or maintaining a Leishmania parasite load below any of the aforementioned levels and/or suppressing Leishmania parasite load at a level below any of the aforementioned levels, in a non-human mammal that suffers or has suffered from leishmaniasis disease, for a period of of at least 3 months, preferably at least 4 months, more preferably at least 5 months. In particularly preferred embodiments, the methods provided are effective in and/or are aimed at and/or are intended for substantially or completely suppressing of *Leishmania* parasite load for a period of at least 3 months, preferably at least 4 months, more preferably at least 5 months. Furthermore, in preferred embodiments, the methods provided are effective in and/or are aimed at and/or are intended for preventing a resurgence of *Leishmania* parasite load for a period of at least 3 months, preferably at least 4 months, more preferably at least 5 months. In particularly preferred embodiments, the methods provided are effective in and/or are aimed at and/or are intended for substantially or completely eradicating *Leishmania* parasite load from the non-human mammal, which typically means that the splenic parasite load is 0, i.e. no parasites are detectable in splenic aspirate, preferably for a period of at least 3 months, more preferably at least 4 months, still more preferably at least 5 months

### Non-human mammal to be treated

As explained herein before, the therapeutic and/or prophylactic methods of the invention comprising the administration of OlPC to a non-human mammal are effective/useful to combat leishmaniasis and/or infection with *Leishmania.*

Hence, preferred embodiments of the invention relate to methods as defined herein, wherein the non-human mammal is a non-human mammal suffering from leishmaniasis or at risk of attracting leishmaniasis or a non-human mammal infected by or at risk of becoming infected by *Leishmania.*

In a preferred embodiment of the invention, the non-human mammal is a non-human mammal that suffers from leishmaniasis and/or that has been infected with *Leishmania* and wherein the non-human mammal is symptomatic, which typically means that the mammal suffers from one or more of the symptoms defined herein before at the start of the treatment. In this case, the mammal may also be said to suffer from leishmaniasis in an active state.

**In** other preferred embodiment of the invention, the non-human mammal is a non-human mammal that suffers from leishmaniasis and/or that has been infected with *Leishmania* and wherein the mammal is asymptomatic. **In** certain preferred embodiments, the non-human mammal has not been symptomatic after attracting leishmaniasis and/or after becoming infected with *Leishmania.* **In** other preferred embodiments, the non-human mammal to be treated in accordance with the invention has substantially or completely recovered from leishmaniasis symptoms at the start of the treatment. **In** the latter case, the mammal may also be said to suffer from leishmaniasis in a dormant or latent state.

**In** preferred embodiments of the invention, the non-human mammal is selected from the group consisting of domesticated animals, i.e. pets or companion animals, including stray pets/animals belonging to species normally considered suitable for domestication, and livestock. In a preferred embodiment, the non-human mammal is selected from the group consisting of dogs; cats; equids, in particular horses; sheep; goats; and rodents, in particular rabbits, still more preferably from the group consisting of dogs, cats and horses, most preferably from the group consisting of dogs.

Hence, in accordance with preferred embodiments of the invention, the non-human mammal has been infected with and/or at risk of becoming infected with *Leishmania infantum.*

In preferred embodiments of the invention, the non-human mammal is suffering from or at risk of contracting a miltefosine treatment resistant leishmaniasis disease variant and/or the non-human mammal is suffering from or at risk of contracting infection with parasites from a miltefosine (treatment) resistant *Leishmania* strain. In particularly preferred embodiments of the invention, the non-human mammal is suffering from or at risk of contracting infection with parasites from a miltefosine (treatment) resistant *L. infantum* strain. In certain particularly preferred embodiments of the invention, the non-human mammal is suffering from or at risk of contracting infection with parasites of strains of *L. infantum* indigenous to areas located in the Americas, including North-America and South-America, more preferably strains of *L. infantum* indigenous to areas located in Mexico, Argentina, Bolivia, Brazil, Chile, Colombia, Ecuador, Guyana, Paraguay, Peru, Suriname, Uruguay, or Venezuela, most preferably strains of *L. infantum* indigenous to areas located in Brazil.

In preferred embodiments of the invention, the non-human mammal suffering from leishmaniasis has received miltefosine treatment or miltefosine and allopurinol combination treatment, and said prior treatment was ineffective in curing the leishmaniasis and/or in substantially alleviating leishmaniasis disease symptoms and/or in preventing a relapse of leishmaniasis disease.

In preferred embodiments of the invention, the non-human mammal suffering from leishmaniasis has received miltefosine treatment or miltefosine and allopurinol combination treatment, and said prior treatment was ineffective substantially reducing *Leishmania* parasite load; and/or in eradicating *Leishmania* parasite load; and/or in preventing *Leishmania* parasite load resurgence.

Embodiments of the invention are also envisaged, wherein the treatment is for prophylactic/preventative purposes and the non-human mammal is a healthy mammal, a mammal not suffering from leishmaniasis, a mammal that has not before been infected with *Leishmania* parasites or a mammal that has suffer from leishmaniasis and/or that has been infected with *Leishmania* parasites and has completely recovered or been cured from such earlies disease and/or infection. Typically, in accordance with this embodiment, the mammal to be treated is at increased risk of attracting leishmaniasis (again) and/or of becoming (re)infected with *Leishmania,* e.g. due to the fact that the animal lives in a (geographical) area located in the Americas, including North-America and South-America, more preferably an area located in Mexico, Argentina, Bolivia, Brazil, Chile, Colombia, Ecuador, Guyana, Paraguay, Peru, Suriname, Uruguay or Venezuela, most preferably an area located in Brazil.

### Treatment regimens

As explained herein before, the therapeutic and/or prophylactic methods of the invention involve the administration, typically the repeated administration, of a composition comprising OlPC, preferably of a composition as defined herein before.

In particularly preferred embodiments of the invention, the treatment comprises the administration of the composition through the enteral route, more preferably through the oral route.

In preferred embodiments of the invention, the treatment comprises the administration, preferably the repeated administration, of the OlPC containing composition at a (OlPC) dosage of at least about 1 mg/kg p.o., more preferably at a dosage of at least about 1.5 mg/kg p.o., at least 1.62 mg/kg p.o., at least 1.63 mg/kg p.o., at least about 1.75 mg/kg p.o., at least about 2 mg/kg p.o., at least about 2.5 mg/kg p.o., at least about 3 mg/kg p.o., at least about 3.5 mg/kg p.o., or at least about 4 mg/kg p.o., and/or at a dosage of about 25 mg/kg p.o. or less, more preferably at a daily of about 20 mg/kg p.o. or less, about 15 mg/kg p.o. or less, about 12 mg/kg p.o. or less, about 10 mg/kg p.o. or less, about 9 mg/kg p.o. or less, about 8 mg/kg p.o., about 7 mg/kg p.o. or less, about 6 mg/kg p.o., about 5 mg/kg p.o. or about 4 mg/kg p.o. or less. In particularly preferred embodiments of the invention, the treatment comprises the administration of OlPC at a dosage of about 1-25 mg/kg p.o., more preferably at a dosage of about 1-8 mg/kg p.o., still more preferably at a dosage of about 2-6 mg/kg p.o., most preferably at a dosage of about 4 mg/kg.

In preferred embodiments of the invention, the treatment comprises the administration, preferably the repeated administration, of the OlPC containing composition at a (OlPC) dose of at least 4.5 mg p.o., more preferably at a (OlPC) dose of at least 4.6 mg p.o., at least 5 mg p.o., at least 8 mg p.o., at least 16 mg p.o., at least 20 mg p.o., at least 25 mg p.o., at least 30 mg p.o., at least 35 mg p.o., at least 40 mg p.o., at least 45 mg p.o., or at least 50 mg p.o., and/or at a (OlPC) dose of 500 mg p.o. or less, more preferably at a (OlPC) dose of 400 mg p.o. or less, 300 mg p.o. or less, 250 mg p.o. or less, 200 mg p.o. or less, 175 mg p.o. or less, 150 mg p.o. or less, 125 mg p.o. or less, 110 mg p.o. or less, 100 mg p.o. or less, 95 mg p.o. or less, 90 mg p.o. or less, 84 mg p.o. or less or 80 mg p.o. or less. In other preferred embodiments, the treatment comprises the administration, preferably the repeated administration, of the OlPC containing composition at a (OlPC) dose within the range of 4.5-500 mg p.o., 25-250 mg p.o., or 50-100 mg p.o..

In particularly preferred embodiments of the invention, the method comprises the administration of OlPC, preferably at the dosages defined here above, at least once every week, at least once every four days, or at least once every two days. In a particularly preferred embodiment of the invention, the method comprises the administration of OlPC, preferably at the dosages defined here above, once daily.

In particularly preferred embodiments of the invention, the treatment comprises the administration of the OlPC containing composition at a daily (OlPC) dosage of at least about 1 mg/kg p.o., more preferably at a daily (OlPC) dosage of at least about 1.5 mg/kg p.o., at least 1.62 mg/kg p.o., at least 1.63 mg/kg p.o., at least about 1.75 mg/kg p.o., at least about 2 mg/kg p.o., at least about 2.5 mg/kg p.o., at least about 3 mg/kg p.o., at least about 3.5 mg/kg p.o., or at least about 4 mg/kg p.o., and/or at a daily dosage of about 25 mg/kg p.o. or less, more preferably at a daily dosage of about 20 mg/kg p.o. or less, about 15 mg/kg p.o. or less, about 12 mg/kg p.o. or less, about 10 mg/kg p.o. or less, about 9 mg/kg p.o. or less, about 8 mg/kg p.o., about 7 mg/kg p.o. or less, about 6 mg/kg p.o., about 5 mg/kg p.o. or about 4 mg/kg p.o. or less. In particularly preferred embodiments of the invention, the treatment comprises the administration of the OlPC containing composition at a daily (OlPC) dosage of about 1-25 mg/kg p.o., more preferably at a daily dosage of about 1-8 mg/kg p.o., still more preferably at a daily dosage of about 2-6 mg/kg p.o., most preferably at a daily dosage of about 4 mg/kg. These dosages may be administered at frequencies/intervals ranging from 4 times a day, meaning that the recited daily dosage is to be divided over four administrations, to once every four days, meaning that 4 daily dosages are to be combined per administration. In preferred embodiments, the methods comprise the administration of OlPC, at the recited daily dosages, twice a day to once every two days. Most preferably, the methods comprise the administration of OlPC, at the recited daily dosages, once daily.

As will be understood by those skilled in the art, based on the present teachings, the daily dosages as recited herein are based on data obtained in dogs (or canine). In the case the mammal to be treated belongs to a different genus, it may be needed to determine appropriate equivalent dosages. It is within the normal skills and capabilities of the skilled person to make such determinations. For instance, appropriate ways of calculating and converting dosages among different genera take account of body surface area in accordance with (e.g.) FDA guidelines concerning the extrapolation of (experimental) animal dosages to (experimental) human dosages.

Preferred embodiments of the invention relate to a therapeutic or curative method, comprising the administration of an effective amount of OlPC, preferably the (once daily) administration of OlPC at the daily dosages defined herein before, for a period of 10 days or more, preferably for a period of 2 weeks or more, more preferably for a period of 3 weeks or more, most preferably for a period of 4 weeks or more, e.g. for a period of 12, 14, 15, 16, 18, 20, 22, 24, 25, 26, 27, 28, 29 or 30 days.

Preferred embodiments of the invention relate to a therapeutic or curative method, comprising a first treatment cycle and a treatment free interval, optionally followed by a second treatment cycle, wherein each treatment cycle comprises the administration of an effective amount of OlPC, preferably the once daily administration of OlPC at the daily dosages defined herein before, for a period of 10 days or more, preferably for a period of 2 weeks or more, more preferably for a period of 3 weeks or more, most preferably for a period of 4 weeks or more, e.g. for a period of 12, 14, 15, 16, 18, 20, 22, 24, 25, 26, 27, 28, 29 or 30 days; and wherein the first treatment cycle is followed by a treatment free interval of at least 3 months, preferably at least 4 months, more preferably at least 5 months, during which the mammal is not administered oleylphosphocholine.

Preferred embodiments of the invention relate to a therapeutic or curative method comprising a plurality of treatment cycles, wherein each treatment cycle comprises the administration of an effective amount of OlPC, preferably the once daily administration of OlPC at the daily dosages defined herein before, for a period of 10 days or more, preferably for a period of 2 weeks or more, more preferably for a period of 3 weeks or more, most preferably for a period of 4 weeks or more, e.g. for a period of 12, 14, 15, 16, 18, 20, 22, 24, 25, 26, 27, 28, 29 or 30 days; and wherein each treatment cycle is followed by a treatment free interval of at least 3 months, preferably at least 4 months, more preferably at least 5 months, during which the mammal is not administered oleylphosphocholine. In particularly preferred embodiments, the method comprises 2, 3,4 ,5, 6, 7, 8, 9 or 10 treatment cycles and an equal number of treatment free interval.

Other preferred embodiments of the invention relate to a prophylactic method comprising the administration of an effective amount of OlPC, once every two days, once every three days, once every four days or once every week, for a period of 4 weeks or more, preferably for a period of 6 weeks or more, 8 weeks or more, 10 weeks or more, 12 weeks or more, 4 months or more, 5 months or more, 6 months or more, 8 months or more 10 months or more or 12 months or more. In these prophylactic treatments the dosage given, per administration may be in accordance with the above defined dosages. It will be understood, by those skilled in the art, based on the present teachings, that longer administration intervals may involve the administration of a (somewhat) larger dose of OlPC (per administration). In some embodiments, the prophylactic methods involve the repeated administration, at the afore-defined frequencies and duration, of OlPC at a dosage of at least about 2 mg/kg p.o., more preferably at a dosage of at least about 3 mg/kg p.o., at least about 4 mg/kg p.o., at least about 4.5 mg/kg p.o., at least about 5 mg/kg p.o., at least about 5.5 mg/kg p.o., at least about 6 mg/kg p.o., at least about 6.5 mg/kg p.o., at least about 7 mg/kg p.o., at least about 7.5 mg/kg p.o., or at least about 8 mg/kg p.o., and/or at a dosage of about 25 mg/kg p.o. or less, more preferably at a daily of about 20 mg/kg p.o. or less, about 17.5 mg/kg p.o. or less, about 15 mg/kg p.o. or less, about 14 mg/kg p.o. or less, about 12 mg/kg p.o. or less, about 11 mg/kg p.o., about 10 mg/kg p.o. or less, about 9 mg/kg p.o., about 8.5 mg/kg p.o. or about 8 mg/kg p.o. or less.

### Kit of Parts

Another aspect of the invention concerns a pharmaceutical kit comprising a package containing a plurality of discrete, OlPC containing dosage forms, such as the unit dosage forms and or the finisched dosage forms defined herein before, and, typically, a leaflet containing printed instructions to repeatedly administer said dosage forms, or a part or fraction thereof, to a non-human mammal in need thereof, via the oral route, so as to attain one or more of the therapeutic and/or prophylactic objectives defined herein elsewhere. In accordance with embodiments of the invention, the pharmaceutical kit comprises at least 5, at least 8, at least 10, at least 12 of at least 15, at least 20, at least 30 or at least 40 of said dosage forms, e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 of said discrete OlPC containing dosage forms. In one embodiment of the invention, the pharmaceutical kit does not comprise any dosage forms comprising a different active agent.

In accordance with preferred embodiments of the invention, the pharmaceutical kit comprises a container, such as a cardboard box, holding one or more blister packs, said one or more blister packs containing a plurality of OlPC containing tablets, preferably a plurality of OlPC containing tablets as defined herein before. In particularly preferred embodiments of the invention, the pharmaceutical kit comprises at least 5, at least 8, at least 10, at least 12 of at least 15, at least 20, at least 30 or at least 40 of said OlPC containing tablets, e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 of said discrete OlPC containing tablets.

In accordance with preferred embodiments of the invention, the pharmaceutical kit comprises a container, such as a cardboard box, holding a plurality of sachets, said sachets containing an OlPC containing granulate, preferably the OlPC containing granulate as defined herein before. In particularly preferred embodiments of the invention, the pharmaceutical kit comprises at least 5, at least 8, at least 10, at least 12 of at least 15, at least 20, at least 30 or at least 40 of said sachets, e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 of said sachets.

In accordance with the invention, the pharmaceutical kit comprises a leaflet inserted into the container, which leaflet may include a description of the form and composition of the unit dosage forms contained in the kit, an indication of the therapeutic indications for which the product is intended, instructions as to how the product is to be used and information and warnings concerning adverse effects and contraindications associated with the use. It will be apparent to those of average skill in the art, based on the information presented herein, that the leaflet will typically contain the information concerning the therapeutic indications, uses, treatment regimens, etc., corresponding to what is described here above in relation to the methods of treatment of the present invention.

### Miscellaneous

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

As used herein, the term "about" when combined with a value refers to plus and minus 10% of the reference value. For example, a length of about 1,000 nanometers (nm) refers to a length of 1,000 nm ± 100 nm.

It is noted that as used herein and in the appended claims, the singular forms "a", "an", and "the" include plural referents unless the context clearly dictates otherwise. Thus, for example, reference to "a polynucleotide" includes a plurality of such polynucleotides and reference to "the polypeptide" includes reference to one or more polypeptides and equivalents thereof known to those skilled in the art, and so forth. It is further noted that the claims may be drafted to exclude any optional element. As such, this statement is intended to serve as antecedent basis for use of such exclusive terminology as "solely", "only" and the like in connection with the recitation of claim elements or use of a "negative" limitation. It should also be noted that the term "or" is generally employed in its broadest sense, that is as meaning "and/or" unless the content clearly dictates otherwise.

The expression "comprise" and variations thereof, such as, "comprises" and "comprising" as used herein should be construed in an open, inclusive sense, meaning that the embodiment described includes the recited features, but that it does not exclude the presence of other features, as long as they do not render the embodiment unworkable

The expressions "one embodiment", "a particular embodiment", "an embodiment" etc. as used herein should be construed to mean that a particular feature, structure or characteristic described in connection with the embodiment is included in at least one embodiment. Thus, the appearances of such expressions in various places throughout this specification do not necessarily all refer to the same embodiment. Furthermore, the particular features, structures, or characteristics may be combined in any suitable manner in one or more embodiments. For example, certain features of the disclosure which are described herein in the context of separate embodiments are also explicitly envisaged in combination in a single embodiment.

In those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the invention are specifically embraced by the present invention and are disclosed herein just as if each and every combination was individually and explicitly disclosed. In addition, all sub-combinations of the various embodiments and elements thereof are also specifically embraced by the present invention and are disclosed herein just as if each and every such sub-combination was individually and explicitly disclosed herein.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### Description of the Figures

Figure 1: design of clinical trial
Figure 2: Mean (SD) of Clinical Scores in Dogs Treated With OlPC or Miltefosine

### Examples

### Example 1: Olevlphosphocholine vs miltefosine for Brazilian Canine Leishmaniasis Introduction

A study was performed aimed at obtaining comparative efficacy and tolerance data of OlPC, 4 mg/kg/day for 28 days, vs the recommended regimen of miltefosine (Milteforan), 2 mg/kg/day for 28 days, in Brazilian dogs naturally infected with *L. infantum.*

### Materials and Methods

### Study Design

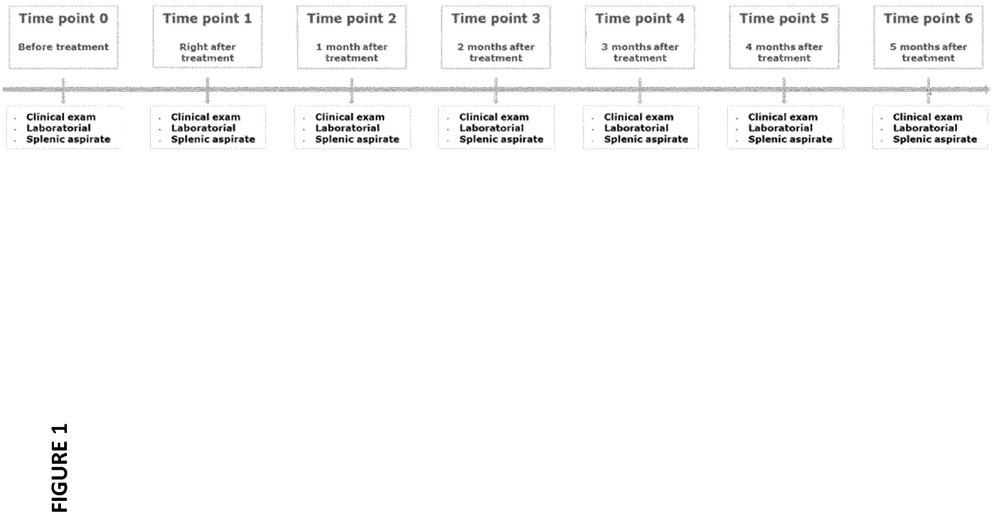

This was a randomized comparison of OlPC to miltefosine, each administered for 28 days, for treatment of canine leishmaniasis with follow up to 6 months after initiation of therapy. The times at which major procedures were performed are shown in Figure 1.

### Animals

Young dogs with clinical signs characteristic of canine visceral leishmaniasis and confirmed infection status according to the diagnostic protocol recommended by the Ministry of Health (rapid test and ELISA) and/or by qPCR were collected by the Zoonoses Control Center of Camaçari (Bahia, Brazil) during routine surveys. Owners signed an informed consent allowing the dog's treatment during a six-month period in a Fiocruz kennel. Dogs were returned to their owners after their treatment.

Dogs were placed in a transport box suitable for their size and taken by car to the Gonçalo Moniz Institute (Salvador-Bahia, Brazil). Upon arriving at the Institute, the animals were housed in separate pens, where they received super premium PREMIER^{®} food in two daily meals, water ad libitum and remained using a deltametrin collar (SCALIBOR^{®}) throughout the study. The dogs were allowed outside the pens for recreation, but not at dawn or dusk when sandflies are most active.

The animals received good general care and appropriate food, for a period of 2 weeks to 1 month. After this period, the animals were evaluated for admission to the protocol. The entrance criteria were age between 1-6 years; weight between 10-30 kg; positive results in ELISA (1.02-1.72 OD units) and qPCR; no prior treatment with antileishmanial drugs; not a pregnant female.

### Initial Procedures

33 dogs were initially admitted to the study. The animals were weighed and evaluated by clinical examination, laboratory tests, and parasitological tests. The clinical examination data were used to construct a clinical score that resulted in 23 parameters being graded 0 (normal), 1 (somewhat abnormal), or 2 (more abnormal) according to Table 1. For each dog, the grades for all clinical parameters were summed to give an overall clinical score for that animal. Laboratory tests included complete blood count (red blood cell count, hemoglobin, mean corpuscular hemoglobin, means corpuscular volume, white cell counts with differential, platelet count) and clinical pathology parameters (ALT, GGT, alkaline phosphatase; BUN and creatinine; albumin, globulin, total protein). Splenic puncture was performed to obtain a splenic aspirate following the technique described by Barrouin et al. (Can spleen aspirations be safely used for the parasitological diagnosis of canine visceral leishmaniosis? A study on assymptomatic and polysymptomatic animals. Vet J. 2006 Mar;171(2):331-9). A portable ultrasound device was used to facilitate the location of the spleen and to reduce the animal's discomfort; 0.5mL of local anesthesia (1% lidocaine hydrochloride) was then administered to the puncture site. The collected sample was kept in the syringe, duly identified and refrigerated, until processing in the laboratory. The material obtained was used to determine parasite load by qPCR.12 Xenodiagnosis was performed as per Borja et al. (Parasite load in the blood and skin of dogs naturally infected by Leishmania infantum is correlated with their capacity to infect sand fly vectors. Vet Parasitol. 2016 Oct 15:229:110-117) in which sandflies feed on the skin of infected dogs and Leishmania transmitted to the sandfly is evaluated by qPCR of the sandfly gut.

**Table 1: Clinical scoring system used in this study**

| **Parameter** | **score = 0** | **score = 1** | **score = 2** |
|---|---|---|---|
| ***Systemic signs*** | | | |
| **Lymph nodes** | Normal | 1 lymph node enlarged | 2 lymph nodes enlarged |
| **Spleen size** | Not palpable | Palpable | ( not applicable) |
| **Mucosal color** | Normal | Hypocolored | Porcelain |
| **Nutritional Status** | Normal | Skinny | Cachectic |
| ***Ocular signs*** | | | |
| **Periocular dermatitis** | Absent | One eye | Both eyes |
| **Conjunctivitis** | Absent | One eye | Both eyes |
| **Uveitis** | Absent | One eye | Both eyes |
| **Vasculitis** | Absent | One eye | Both eyes |

| ***Dermatological signs*** | | | |
|---|---|---|---|
| **Crust on the tip of the ear** | Absent | One ear | Both ear |
| **Onychogryphosis** | Absent | Nails with discreet augmentation | Expressive nail augmentation |
| **Presence of ulcers** | Absent | Present | (not applicable) |
| **Depigmentation** | Absent | Present <1/3 of muzzle tip | Present >1/3 of muzzle tip |
| **Hyperkeratosis** | Absent | Present <1/3 of muzzle tip | Present >1/3 of muzzle tip |
| **Alopecia** | Absent | Focally present in some body areas | Disseminated - >1/3 of body |
| **Furfuraceous dermatitis** | Absent | Focally present in some body areas | Disseminated - >1/3 of body |
| **Nodular dermatitis** | Absent | Focally present in some body areas | Disseminated - >1/3 of body |
| **Papular dermatitis** | Absent | Focally present in some body areas | Disseminated - >1/3 of body |
| **Pustular dermatitis** | Absent | Focally present in some body areas | Disseminated - >1/3 of body |

| ***Other signs*** | | | |
|---|---|---|---|
| **Mucous lesions** | Absent | Present | (not applicable) |
| **Arthritis/limping** | Absent | Present | (not applicable) |
| **Epistaxis** | Absent | Present | (not applicable) |
| **Diarrhea** | Absent | Present | (not applicable) |
| **Vomiting** | Absent | Present | (not applicable) |
| **Fever** | Absent | Present | (not applicable) |

### Treatment

After the initial evaluation was complete, animals were randomized between OlPC, 4 mg/kg/day orally for 28 days, and miltefosine, 2 mg/kg/day orally for 28 days.

The dog OlPC dose level was chosen because 4 mg/kg was the no-observed adverse effect level in a canine toxicology study and was also the dose level used (within liposomes) in the prior Hernandez canine study. OlPC was manufactured according to GMP by Oblita LLC and donated to the study. For this study, OlPC was formulated as an aqueous solution of OlPC (2 wt.%) and vitamin E (0.02 wt.%) in demineralized water (freshly prepared every week and stored under refrigeration).

The miltefosine dose level was 2 mg/kg/day, lower than the OlPC dose level. This dose level was chosen because it is the recommended dose level for Milteforan. Milteforan was purchased from Virbac [Carros, France].

Initially, 33 dogs were randomized 17 to OlPC and 16 to miltefosine. However in the course of the study, 3 owners of miltefosine dogs requested the return of their animals. The 3 removed miltefosine dogs were replaced per protocol with 3 dogs with the same treatment assignment (miltefosine). Overall, this was a study of 17 OlPC dogs randomized vs 13 miltefosine dogs plus 3 unrandomized miltefosine dogs for a total of 17 OlPC dogs vs 16 miltefosine dogs.

### Follow up

At 1, 2, 3, 4, 5, and 6 months after starting treatment (ie, 0, 1, 2, 3, 4 and 5 months after the end of therapy. See Figure 1), the clinical examination was repeated to construct a clinical score and blood was collected for clinical pathology. At 6 months after starting treatment, splenic aspiration was repeated to obtain a splenic parasite load and xenodiagnosis was repeated to obtain a skin parasite load. The staff responsible for the clinical evaluation, laboratory studies, and parasitological studies were blinded to the treatment assignment of the animals being evaluated.

### Pharmacokinetics

Blood samples for pharmacokinetics were collected from eight OlPC animals at pre-dose; 1, 4, 8, 12, and 24 hrs after dosing on Day 1; and 1, 4, 8, 12, and 24 hrs after dosing on Day 28. On Days 7, 14, and 21, one blood sample was drawn before OlPC administration to calculate trough levels. The blood was frozen and shipped at -60°C or below to Quinta Analytica, Czech Republic, where total plasma concentration of OlPC was performed by liquid chromatography with mass spectrometric detection (LC-MS/MS). The systemic exposure to OlPC was assessed by non-compartmental analysis.

### Endpoints

The primary endpoint was reduction in the clinical score. Secondary endpoints were reduction of parasite load in splenic aspirates and in skin via xenodiagnoses, values of clinical pathology, and plasma levels of OIPC.

### Statistical Analysis

Clinical scores were compared by the Student's T-test. Splenic parasite values were compared by the Mann-Whitney U-test.

### Results

### Clinical Scores

At entrance, the clinical score for OlPC was [mean (SD)] 10.1 (5.6) and for miltefosine was 7.7 (4.5) (See Table 2), a non-significant difference. Since all but a rare animal had a score = 0 for 8 parameters (nodular dermatitis, pustular dermatitis, mucosal lesions, arthritis/limping, epistaxis, diarrhea, vomiting, and fever), the clinical scores reflected the contributions of the 15 other clinical parameters: the systemic signs of lymph node enlargement, splenic enlargement, decreased mucosal color, decreased nutritional status; the ocular signs of periocular dermatitis, conjunctivitis, uveitis, and vasculitis; and the dermatological signs of ear crusts, onychogryphosis, ulcers, depigmentation, hyperkeratosis, alopecia, and furfuraceous dermatitis. For 13 of these 15 parameters, the values for OlPC and for miltefosine were non-significantly different (See Table 2) The largest contributors to the clinical score were enlarged lymph nodes (mean score for all animals = 1.51), onychogryphosis (mean score = 1.36), ear crusts (mean score = 1.33), and furfuraceous dermatitis (mean score = 0.76).

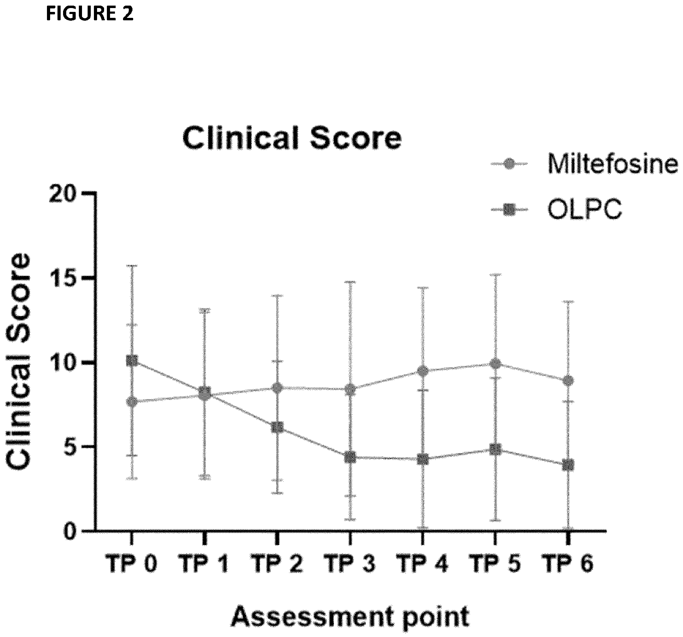

At the end of the 1-month period of therapy, the mean clinical scores had only slightly changed: 8.2 for OlPC and 8.1 for miltefosine (See Figure 2)]. At 1 month after therapy, mean clinical scores had decreased for OlPC (6.2) but had not decreased for miltefosine (8.5). [Fig 3]

By 2 months after therapy, the OlPC score had dramatically decreased whereas the score for miltefosine was essentially unchanged. (See Figure 2) The scores were 4.4 (3.7) for OlPC versus 8.4 (6.3) for miltefosine which was just statistically significant (P = 0.04).

At 3 months after therapy (month 4 of the trial), OlPC animals had a slightly lower clinical score (4.3) than at 2 months whereas miltefosine animals had a somewhat raised value (9.5) compared to 2 months. (See Figure 2 and Table 2) (P=0.003). The marked statistical difference between overall clinical scores was primarily due to statistical differences in lymph nodes, ear crusts, onychogryphosis, and depigmentation (Table 2).

At 4 months after therapy, both OlPC scores and miltefosine scores had leveled off [Figure 3]. The mean clinical score for OlPC was 4.9 (4.2) and the score for miltefosine was 9.9 (5.3) [P = 0.005].

At the end of the experiment at 5 months after therapy (month 6 of the trial), OlPC and miltefosine scores were lower than at 4 months after therapy (See figure 2 and Table 2). The overall OlPC clinical score was 3.9 (3.8) and the overall score for miltefosine was 8.9 (4.7) [P =0.002] As before, the major drivers for the statistical difference between OlPC and miltefosine were lymph nodes and ear crusts which suggests that both visceral and cutaneous parameters contributed to the statistically improved OlPC score relative to miltefosine. For OlPC, the clinical score at the end of the study was statistically lower than at the beginning of the study: P < 0.001.

Review of Figure 3 indicates that OlPC treatment led to an almost straight-line decrease in the clinical score from pre-therapy to 2 months after the end of therapy, by which time the mean clinical score had decreased to 44% of the entrance score, with then little change from that time point until the end of the study at 5 months after the end of therapy. In contrast, miltefosine treatment never led to a decrease in the clinical score.

**Table 2: Clinical scores at month as 0, 4 and 6**

| **Month** | **0** | **0** | **4** | **4** | **6** | **6** |
|---|---|---|---|---|---|---|
| **Treatment Group** | **Milt** | **OIPC** | **Milt** | **OIPC** | **Milt** | **OIPC** |
| **Overall Clinical Score** | 7.7 (4.5) | 10.1(5.6) | 9.5(4.9)# | 4.3(4.1)# | 8.9 (4.7)# | 3.9 (3.8)# |
| ***Systemic Signs*** | | | | | | |
| **Lymph nodes** | 1.3(0.6) | 1.71(0.59) | 1.63 (0.62)# | 0.76 (0.9)# | 1.7(0.6)* | 0.94(0.93)* |
| **Spleen size** | 0.5(0.52) | 0.41(0.51) | 0.44(0.51) | 0.41(0.51) | 0.44(0.51) | 0.38(0.50) |
| **Mucosal color** | 0.12(0.34) | 0.29(0.47) | 0.25(0.45) | 0.12 (0.33) | 0.25(0.45) | 0.19(0.40) |
| **Nutritional Status** | 0.06(0.25)! | 0.47(0.62)! | 0(0) | 0.06(0.24) | 0.06(0.25) | 0(0) |

| ***Ocular signs*** | | | | | | |
|---|---|---|---|---|---|---|
| **Periocular dermatitis** | 0.25(0.68) | 0.53(0.87) | 0.25(0.68) | 0 (0) | 0.19(0.54) | 0.13(0.5) |
| **Conjunctivitis** | 0.25(0.68) | 0.47(0.87) | 0.38(0.81) | 0.18 (0.53) | 0.50(0.89)! | 0(0)! |
| **Uveitis** | 0.12(0.5) | 0.12(0.33) | 0.50(0.89) | 0.29(0.69) | 0.25(0.68) | 0.13(0.50) |
| **Vasculitis** | 0.19(0.54) | 0.41(0.71) | 0.50(0.89) | 0.24 (0.66) | 0.69(0.87)! | 0.13(0.50) |

| ***Dermatological signs*** | | | | | | |
|---|---|---|---|---|---|---|
| **Crust on tip of ear** | 1.19 (.98) | 1.47 (0.87) | 1.44(0.81)# | 0.41(0.71)# | 1.5 (0.82)# | 0.5(0.82)# |
| **Onychogryphosis** | 1.25(0.93) | 1.47(0.72) | 1.38 (0.62)! | 0.82(0.81)! | 1.1(0.69) | 0.56 (0.81) |
| **Ulcers** | 0.31(0.48) | 0.24(0.44) | 0.06 (0.25) | 0(0) | 0(0) | 0(0) |
| **Depigmentation** | 0.69(0.6) | 0.41(0.71) | 0.63(0.62)# | 0.06(0.24)# | 0.31(0.48) | 0.06(0.25) |
| **Hyperkeratosis** | 0.44(0.73) | 0.53(0.72) | 0.75(0.86) | 0.35(0.70) | 0.62(0.81) | 0.28(0.58) |
| **Alopecia** | 0.25(0.58)! | 0.76(0.83)! | 0.31(0.6) | 0.18 (0.53) | 0.44(0.73) | 0.28(0.58) |
| **Furfuraceous dermatitis** | 0.75(0.86) | 0.76(0.90) | 0.94(0.77) | 0.41 (0.62) | 0.81(0.83) | 0.44(0.73) |
| #OlPC vs Milt P-value <0.01 | | | | | | |
| *OlPC vs Milt P-value= 0.01 | | | | | | |
| **OlPC vs Milt P-value=0.02 | | | | | | |
| ! OlPC vs Milt P-value = 0.02-0.05 | | | | | | |

### Parasitology

The very large difference between mean and median parasite counts for both OlPC and miltefosine animals (See Table 3) reflects the non-normal distribution for this parameter. For example, at study entrance the mean and median counts in OlPC animals were 881 thousand and 353 thousand, respectively, and for miltefosine, the mean and median were 2,490 thousand and 275 thousand, respectively. Differences between OlPC parasites and miltefosine parasites were therefore investigated by non-parametric statistics. At study entrance, there was no statistical difference (Mann-Whitney U-test) between the groups (P = 0.56) and 1 animal in each treatment group was without detected parasites in the splenic sample. For months 1-4, median parasites in the OlPC group were "0" because 9-to-12 of the 17 animals did not demonstrate parasites, whereas only 3-to-5 of the 16 animals in the miltefosine group were without demonstrable parasites. On month 5, median OlPC parasites were 5,799 compared to median parasites of 174,118 in the miltefosine group. Splenic parasites were statistically less in the OlPC group compared to the miltefosine group at months 1, 2, 3, and 5. At the end of the experiment on month 6, OlPC splenic parasites were 21,129 compared to 60,974 in the miltefosine group, (P=0.13). For OlPC, the splenic parasitological score at the end of the study was statistically lower than at the beginning of the study (Z = 2.57, P =0.01), whereas for miltefosine the change in splenic parasites was not statistically lower (Z=0.98, P = 0.33).

**Table 3: Splenic parasite scores**

| **Month** | **0** | **1** | **2** | **3** | **4** | **5** | **6** |
|---|---|---|---|---|---|---|---|
| | | | | | | | |
| **OIPC mean** | 881,017 | 12,710 | 66,738 | 1,095,398 | 1,258,703 | 1,381,004 | 451,578 |
| **OIPC median** | 353,965 | 0 | 0 | 0 | 0 | 5799 | 21,129 |
| **OIPC range*** | 0-5,547 | 0-174 | 0-986 | 0-18,251 | 0-20,888 | 0-22,964 | 6,179 |
| **#animals with no parasites** | 1 | 12 | 9 | 9 | 9 | 7 | 6 |
| | | | | | | | |
| **Milt mean** | 2,490,948 | 432,821 | 1,197,765 | 1,850,759 | 240,048 | 1,074,995 | 1,124,239 |
| **Milt median** | 275,864 | 1,375 | 118,065 | 161,861 | 11,777 | 174,118 | 60,974 |
| **Milt range*** | 0-15,824 | 0-5,574 | 0-15,159 | 0-18,524 | 0-2,375 | 0-11,867 | 0-10,834 |
| **#animals with no parasites** | 1 | 5 | 3 | 3 | 4 | 3 | 3 |
| | | | | | | | |
| **Mann-Whitney Z score** | 0.576 | 2.3 | 2.6 | 2.6 | 1.6 | 2.45 | 1.53 |
| **Mann-Whitney P-value** | 0.56 | 0.022 | 0.008 | 0.008 | 0.11 | 0.014 | 0.13 |
| Values are parasites/ml parasite aspirate | | | | | | | |
| *Values are 1000parasites/ml parasite aspirate | | | | | | | |
| There were 17 OIPC animals and 16 Miltefosine (Milt) animals at each time point, except for month 6 where there were 16 OIPC animals. | | | | | | | |

### Xenodiagnosis

Xenodiagnoses before therapy and at the end of study was accomplished on 9 of the 17 OlPC dogs and 7 of the 16 miltefosine dogs. For OlPC, values at the end of the study were roughly the same as before therapy for 6 dogs, had increased for 1 dog, and had decreased for 2 dogs. Similar results were seen for miltefosine dogs: values at the end of the study were comparable to before therapy for 5 dogs, had increased for 1 dog, and had decreased for 1 dog.

### Death

One dog in the OlPC group died between the 4th month post-treatment and the end of the study 5 months post treatment. In accord with the usual cause of death in canine leishmaniasis, the death was due to kidney failure. Histopathological investigation indicated diffuse tubular necrosis, and also deposition of hyaline material presumably immunoglobulins in the mesangium. At pre-treatment, creatinine and BUN were within normal limits (1.0 mg/dL and 44 mg/dl, respectively). By 1 month after the end of therapy, creatinine and BUN had risen to 2.7 mg/dL and 82 mg/dL, respectively. Just before death at 4 months after therapy, creatinine and BUN were 14.2 mg/dL and 347 mg/dL, respectively. Splenic parasitology revealed very few parasites pre-treatment (102 parasites/ml splenic aspirate) which fell to 0 parasites/ml aspirate by the end of therapy and remained at 0 parasites/ml aspirate for the rest of the study. The few parasites at entrance coupled with the subsequent complete elimination of parasites leads us to hypothesize that immune-complex formation and deposition probably present at study entrance rather than OlPC parasitological failure led to the death of this animal.

### Laboratory parameters

Prior to treatment, OlPC dogs initially weighed 18 (11) kg which increased to 22 (12) kg at month 6. Miltefosine dogs initially weighed 16 (5) kg which rose to 18 (6) kg at month 6, the end of the study.

Pre-treatment OlPC dogs were anemic pre-treatment (29 %: normal range =37-55 %) and improved to 34 % at month 6. Miltefosine animals were slightly anemic pre-treatment (hematocrit = 35%) and stayed that way (hematocrit = 34 %) at month 6.
For OlPC animals, globulins were 6.2 g/dL (normal range = 2.7-4.4 g/dL) at entrance and decreased to 5.1 g/dL at month 6. Globulins were also increased pre-treatment in the miltefosine animals (5.7 g/dL) and were similarly high at month 6 (5.7 g/dL).

Creatinine pre-treatment was 1.2 (0.5) mg/dL for OlPC animals and 1.1 (0.5) mg/dL for miltefosine animals (normal range: 0.5-1.5 mg/dL). For miltefosine on month 6, creatinine had not changed: 1.1 (0.4) mg/dL. OlPC creatinine values on month 6 were 1.57 (0.6), which is borderline higher than the 1.50 mg/dL upper limit of normal, with BUN = 48 mg/dL (normal values: < 60 mg/dL).

### Pharmacokinetics

On day 1, T max was reached at 7 hr, Cmax = 4.5 ug/ml, and AUC (0-24) was 83 ug/ml x hr. On day 28, T max was similarly reached at 8.5 hr, Cmax = 13.3 ug/ml, and AUC (0-24) was 299 ug/ml x hr.

The accumulation factor based on Cmax and on AUC was 3-to-4. If the overall accumulation factor were to be 3.4, the half-life of OlPC in dog can be calculated to be 48 hr.

### Discussion

A randomized comparison of the miltefosine analog OlPC to miltefosine itself was performed, the oral standard of care for Leishmania infections, in Brazilian dogs. At entrance, the primary clinical signs were lymph node enlargement, onychogryphosis, ear crusts, and furfuraceous dermatitis. Creatinine was normal but globulins were raised. This data suggests that the animals entered with grade 1-to-2 disease on the LeishVet scale.

Both drugs were administered for 28 days. OlPC was orally administered at 4 mg/kg/day, the dose level of OlPC (as liposomal-OlPC) used in a previous canine study, whereas miltefosine formulated as Milteforan was orally administered at 2 mg/kg/day, the approved canine regimen of Milteforan which is similar to the approved human level of 2.5 mg/kg/day. OlPC pharmacokinetic analysis revealed a T ½ of approximately 48 hrs and Cmax at steady state =13 ug/ml. Miltefosine plasma levels in dog are to our knowledge unreported. In humans given 1.8 mg/kg/day, Cmax = 37 ug/ml.4 Since 1.8 mg/kg/day in human equates to 3.2 mg/kg/day in dog, dogs that received miltefosine 2.0 mg/kg/day might be expected to have a miltefosine Cmax= (2.0/3.2 x 37) = 23 ug/ml. If so, although OlPC was administered at twice the dose of miltefosine, plasma levels of OlPC would be approximately half that of miltefosine. The discrepancy between relative administered dose and relative plasma levels may be due to miltefosine's long half-life in dog of 159 hrs.

The primary endpoint for this study was reduction in the clinical score, a composite variable composed of 23 individual clinical parameters of which 15 were abnormal at entrance in the present study. Higher scores signify more extensive and/or more severe disease. The clinical score was computed for each treatment group at the beginning of therapy (month 0), at the end of therapy (month 1), and at each month through 5 months after the end of therapy (month 6 of the study). For OlPC, the mean clinical score was 10.1 pre-therapy, decreased by the end of therapy monotonically through month 3, then leveled off to the end of the study where the score was 3.9, a 61% decrease from pre-therapy [Table 2 and Figure 2] For miltefosine, the mean clinical score was 7.7 pre-therapy and 8.9 at study month 6, a 13% increase. [Table 2 and Figure 2] At each of 3, 4, 5, and 6 months of the trial, the OlPC clinical score was statistically lower than the miltefosine clinical score [Figure 3 and Table 2]

Miltefosine, alone or in combination with allopurinol, has been prospectively evaluated several times (see Table 4). In addition to the prospective studies in Table 4, Gizzarelli reported a retrospective study of 173 Italian dogs who were treated with miltefosine (2 mg/kg/day for 28 days) plus allopurinol (10 mg/kg twice a day for 2-to-12 months) and 30 dogs (17%) required a second treatment.21

**Table 4: clinical responses to miltefosine alone or with allopurinol in studies with > 1 month follow up**

| **endemic region** | **#dogs** | **Milt dose** | **Allo dose** | **Clinical score at preRx** | **Clinical score at follow up (month) / %preRx** | **Author (year)** |
|---|---|---|---|---|---|---|
| **Mediterranean** | 96 | 2MKD x 28D | [none] | 15 | 5(1) / 31% | Woerly (2009) |
| **Brazil** | 35 | 2MKD x 28D | [none] | 16 | 5 (2) / 31% | Nogueira (2019) |
| **Brazil** | 10 | 2MKD x 28D | [none] | 6 | 2.5 (2) / 42% | Ramos (2023) |
| **Southern Europe** | 37 | 2MKD x 28D | 10 mg/kg BID for whole study | 19 | 2 (6) / 10% | Miro (2009) |
| **Italy** | 28 | 2MKD x 28D | 10 MKD for whole study | 5.2 | 0.9 (6) / 17% | Manna (2015) |
| **Brazil** | 15 | 2MKD x 28D | 10 mg/kg BID x 28D | 6 | 1 (12) / 17% | Vaz (2023) |

The literature summarized in Table 4 suggests that miltefosine alone reduced the clinical score by 60-70% in short-term studies with 1-2 months follow-up post-treatment, and that Miltefosine plus allopurinol reduced the clinical score by 80-90% over 6-12 months. In spite of the suggestion that miltefosine's efficacy needs to be augmented by concomitant allopurinol therapy, the literature does suggest some efficacy for miltefosine alone, a conclusion which differs from the data of the present study, in which miltefosine had no beneficial effect on the clinical score.

Canine leishmaniasis is a multi-system disease with visceral and cutaneous signs. Parasitology was investigated to see if visceral parasites as represented by splenic aspirates and cutaneous parasites as represented by xenodiagnosis correlated with the clinical differences between OlPC and miltefosine. In the spleen, OlPC animals had statistically less parasites than did miltefosine animals on months 1, 2, 3, and 5 of the study, in part due to the larger number of animals in whom 0 parasites were found in the OlPC group (6-to-12 animals per month for months 1-6) compared to the miltefosine group (3-5 animals for those months). (See Table 3). xenodiagnostic data was unfortunately non-contributory, perhaps because only half the animals could be evaluated both before therapy and at the end of the study,

A possible clinical pathological abnormality in OlPC dogs is suggested by the elevation of mean creatinine values from 1.2 mg/dL pre-Rx to 1.57 mg/dL, slightly above the 1.50 mg/dL normal limit for this laboratory, at the end of the study. The rise in creatinine was not accompanied by a rise in BUN and should be further investigated in future studies.

Overall, the superior clinical effect of OlPC to miltefosine in this canine study conforms to the superiority of OlPC to miltefosine in mouse and hamster reports and primes OlPC to be developed as an oral treatment for canine and human leishmaniasis.

### Example 2: Prophylactic use of Oleylphosphocholine in hamsters

### Introduction

To explore the potential of OlPC as a prophylactic treatment, an experiment in the hamster *L. infantum* VL model was performed. To test prophylactic activity of OlPC against L. infantum infection, the drug was given orally as a single high dose of 100 mg/kg to hamsters prior to intravenous infection with *L. infantum* amastigotes. The amastigotes surviving exposure to the drug establish infection and divide in target organs until day 21, on which the animals are euthanized and target organ parasitemia is determined. This infection model is a research tool which does not mimic natural infection with promastigotes.

### Experiment A: High dose infection, single drug dosing at different times pre-infection

- Infective dose: 2x10⁷ amastigotes intravenously, 7 hamsters per group.
- Treatment: Oral, single-dose administration, 100 mg/kg OlPC (aqueous solution)
- Dosing regimen: Day -7, -4, -1 and day 0 (6 hours before infection)
- Evaluation criteria: Parasite burden in target organs (liver, spleen, bone marrow) by microscopic evaluation on day 21 post infection.

Results are summarized in table A. It can be seem that OlPC, given as 100 mg/kg single-dose orally, had a measurable effect on Leishmania amastigote burden on day 21 when given up to 7 days before infection (observed effect in spleen > bone marrow > liver). When given 4 days before infection, OlPC reduced the amastigote burden by an average of ±50% in target organs on day 21. When given on Day 0 (6 hours prior to infection), OlPC reduced parasitemia on day 21 by 92-99%.

**Table A. Experiment A - High dose infection, % reduction of organ amastigote burden compared to vehicle control on day 21 post infection.**

| ***Group*** | ***Reduction in parasitemia (%)*** | | | |
|---|---|---|---|---|
| | ***Liver*** | ***Spleen*** | ***Bone Marrow*** | ***AVERAGE**** |
| *OIPC Day -7* | ***0*** | ***40*** | ***10*** | ***17*** |
| *OIPC Day -4* | ***22*** | ***77*** | ***57*** | ***52*** |
| *OIPC Day -1* | ***55*** | ***82*** | ***67*** | ***68*** |
| *OIPC Day 0* | ***92*** | ***99*** | ***94*** | ***95*** |
| * average reduction for the three organs | | | | |

When given prophylactically as a single oral dose, OlPC greatly reduces *L. infantum* organ burden in hamsters. Assuming that the day of dosing has an impact on total drug exposure, the observed prophylactic effect is dose-dependent. Reduction of parasitemia could have a significant impact on the development of symptomatic disease (disease prevention).

### Example B: Infection dose titration, single drug dosing at 6 hours pre-infection

- Infective dose: High-dose (2x107 amastigotes intravenously), Mid-dose (2x106 amastigotes intravenously) and Low-dose infection (2x105 amastigotes intravenously), 6 hamsters per group.
- Treatment: Oral, single-dose administration of 100 mg/kg OlPC (aqueous solution)
- Dosing regimen: Dosing at day 0 (6 hours before infection)
- Evaluation criteria: Parasite burden in target organs (liver, spleen, bone marrow) by microscopic evaluation on day 21 post infection

Results are summarized in table B. It can be seen that, when given on Day 0, OlPC reduced parasitemia on day 21 by >80% for the high infective dose (2x10⁷), and by >90% for mid (2x10⁶) and low infection dose *(2x10⁵*

**Table B: Experiment B - Infection dose titration, % reduction of organ amastigote burden compared to vehicle control on day 21 post infection.**

| **Group** | **Reduction in parasitemia (%)** | | |
|---|---|---|---|
| | **Liver** | **Spleen** | **Bone Marrow** |
| High-dose infection | 85 | 94 | 80 |
| Mid-dose infection | 96 | 94 | 96 |
| Low-dose infection | 92 | 100 | 100 |

The infective dose has an impact on the capacity of the parasites to establish infection in hamsters, as parasitemia was not detected in all vehicle-treated hamsters following low dose infection. Although prophylaxis with OlPC only marginally reduced the percentage of infected animals after low dose infection, our observations do suggest that OlPC is more potent at reducing organ parasitemia when the infective dose is lower. This implies that in the context of a more natural infection model, where only low amounts of promastigotes are inoculated in the host, a safe dose would be able to prevent animals from getting infected.

### Discussion

OlPC shows potential as a prophylactic drug when given as a single dose before infection in terms of reduction of parasitemia. The long half-life and good oral availability make it a suitable drug candidate for oral dosing given in a weekly, bi-weekly or possible monthly administration regimen. The main limitation of the current experimental set-up is that it does not mimic a natural infection setting. Massive amounts of Leishmania amastigotes are directly injected into the bloodstream, which is likely followed by rapid sequestration of the parasites in the liver and establishment of infection. In this set-up it may be extremely difficult for any drug to actually prevent infection, and that OlPC is performing remarkably well.

## Claims

1. Veterinary (pharmaceutical) composition comprising oleylphosphocholine for use in a therapeutic or prophylactic method of treating leishmaniasis in a non-human mammal, the method comprising the oral administration of an effective amount of oleylphosphocholine to said mammal.

2. Veterinary (pharmaceutical) composition for use according to claim 1, wherein the veterinary (pharmaceutical) composition comprises a granulate comprising a mixture of olelyl phosphocholine, an anti-oxidant, a filler and, optionally, and/or one or more further granulation excipients.

3. Veterinary (pharmaceutical) composition for use according to claim 2, wherein the granulate is a granulate obtainable by a melt-agglomeration process, preferably a hot-melt extrusion process, or a wet granulation process, preferably a high-shear wet granulation process.

4. Veterinary (pharmaceutical) composition for use according to claim 2 or 3, wherein:
- the antioxidant is selected from the group consisting of alpha tocopherol, vitamin E, alpha tocopherol acetate, ascorbic acid, butylated hydroxyanisole (BHA), butylated hydroxytoluene (BHT), tert-butylhydroquinone (TBHQ), propyl gallate and ethoxyquin and mixtures thereof, most preferably from alpha tocopherol and Vitamin E; and
- the filler is selected from the group consisting of calcium carbonate, calcium phosphate (dibasic), calcium phosphate (tribasic), calcium sulphate, cellulose, microcrystalline cellulose, microcrystalline silicified cellulose, powdered cellulose, dextrates, dextrose, fructose, lactitol, lactose monohydrate, magnesium carbonate, maltitol, maltodextrin, maltose, mannitol, sodium chloride, sorbitol, starch, pregelatinized starch, sucrose, compressible sugar, sugar spheres, talc, xylitol, silicon dioxide, and mixtures thereof.

5. Veterinary (pharmaceutical) composition for use according to any one of claims 2-4, wherein the concentration of oleyl phosphocholine in the OlPC containing granulate is between 10 and 50 wt.%, preferably between 25 and 35 wt.%.

6. Veterinary (pharmaceutical) composition for use according to any one of claims 1-5, wherein the non-human mammal is a domesticated animal or a companion animal, preferably a domesticated or companion animal selected from the group consisting of dogs, cats, equids, sheep, goats and rabbits.

7. Veterinary (pharmaceutical) composition for use according to any one of the preceding claims 1-6, wherein the non-human mammal is a dog.

8. Pharmaceutical or veterinary composition for use according to claim 7, wherein the effective amount is less than 12 mg/kg p.o. daily, preferably 8 mg/kg p.o. daily, preferably 1-8 mg/kg p.o. daily, more preferably 2-6 mg/kg p.o. daily.

9. Veterinary (pharmaceutical) composition for use according to claim 7 or 8, wherein the method comprises the once daily administration of an effective amount of oleylphosphocholine for a period of 10 days or more, preferably for a period of 2 weeks or more, more preferably for a period of 3 weeks or more, most preferably for a period of 4 weeks or more.

10. Veterinary (pharmaceutical) composition for use according to any one of the preceding claims, wherein the non-human mammal is suffering from or at risk of contracting a miltefosine treatment resistant leishmaniasis disease variant.

11. Veterinary (pharmaceutical) composition for use according to any one of the preceding claims, the non-human mammal is suffering from or at risk of contracting infection with miltefosine resistant *Leishmania* parasites.

12. Veterinary (pharmaceutical) composition for use according to any one of the preceding claims, wherein the non-human mammal suffering from leishmaniasis has received miltefosine treatment or miltefosine and allopurinol combination treatment, wherein said prior treatment was ineffective in curing the leishmaniasis and/or in substantially alleviating leishmaniasis disease symptoms and/or in preventing a relapse of leishmaniasis disease.

13. Veterinary (pharmaceutical) composition for use according to any one of the preceding claims, wherein the non-human mammal suffering from leishmaniasis has received miltefosine treatment or miltefosine and allopurinol combination treatment, wherein said prior treatment was ineffective substantially reducing *Leishmania* parasite load; and/or in eradicating *Leishmania* parasite load; and/or in preventing *Leishmania* parasite load resurgence.

14. Veterinary (pharmaceutical) composition for use according to any one of the preceding claims, wherein the treatment results in substantially or complete suppression of *Leishmania* parasite load for a period of at least 3 months, preferably at least 4 months, more preferably at least 5 months.

15. Veterinary (pharmaceutical) composition for use according to any one of the preceding claims, wherein the treatment prevents resurgence of *Leishmania* parasite load for a period of at least 3 months, preferably at least 4 months, more preferably at least 5 months.

16. Veterinary (pharmaceutical) composition for use according to any one of the preceding claims, wherein the treatment results in substantially or complete suppression of leishmaniasis disease symptoms for a period of at least 3 months, preferably at least 4 months, more preferably at least 5 months.

17. Veterinary (pharmaceutical) composition for use according to any one of the preceding claims, wherein the treatment results in a leishmaniasis disease free interval of at least 3 months, preferably at least 4 months, more preferably at least 5 months.

18. Veterinary (pharmaceutical) composition for use according to any one of the preceding claims, wherein the method comprising a first treatment cycle and a treatment free interval, optionally followed by a second treatment cycle, wherein each treatment cycle comprises the once daily administration of an effective amount of oleylphosphocholine for a period of 2-6 weeks, preferably a period of about 4 weeks, and wherein the first treatment cycle is followed by a treatment free interval of at least 3 months, preferably at least 4 months, more preferably at least 5 months, during which the mammal is not administered oleylphosphocholine.

19. Veterinary (pharmaceutical) composition for use according to any one of the preceding claims, wherein the method comprising a plurality of treatment cycles, wherein each treatment cycle comprises the once daily administration of an effective amount of oleylphosphocholine for a period of 2-6 weeks, preferably a period of about 4 weeks, and wherein each treatment cycle is followed by a treatment free interval of at least 3 months, preferably at least 4 months, more preferably at least 5 months, during which the mammal is not administered oleylphosphocholine.

20. Veterinary (pharmaceutical) composition comprising oleylphosphocholine for use in a method of preventing a resurgence of *Leishmania* parasite load and/or maintaining a low *Leishmania* parasite load and/or suppressing *Leishmania* parasite load in a non-human mammal that suffers or has suffered from leishmaniasis disease, the method comprising the oral administration of an effective amount of oleylphosphocholine to said mammal.

21. Veterinary (pharmaceutical) composition comprising oleylphosphocholine for use in a method of preventing leishmaniasis disease relapse in a non-human mammal, the method comprising the oral administration of an effective amount of oleylphosphocholine to said mammal.

22. Use of oleylphosphocholine in the manufacture of a veterinary (pharmaceutical) composition for the therapeutic or prophylactic treatment of leishmaniasis in a non-human mammal, the method comprising the oral administration of an effective amount of oleylphosphocholine to said mammal.

23. Use of oleylphosphocholine in the manufacture of a veterinary (pharmaceutical) composition for preventing a resurgence of *Leishmania* parasite load and/or maintaining a low *Leishmania* parasite load and/or suppressing *Leishmania* parasite load in a non-human mammal that suffers or has suffered from leishmaniasis disease, the method comprising the oral administration of an effective amount of oleylphosphocholine to said mammal.

24. Use of oleylphosphocholine in the manufacture of a veterinary (pharmaceutical) composition for preventing leishmaniasis disease relapse in a non-human mammal, the method comprising the oral administration of an effective amount of oleylphosphocholine to said mammal.

25. Method for the therapeutic or prophylactic treatment of leishmaniasis in a non-human mammal, the method comprising the oral administration of an effective amount of oleylphosphocholine to said mammal.

26. Method of preventing a resurgence of *Leishmania* parasite load and/or maintaining a low *Leishmania* parasite load and/or suppressing *Leishmania* parasite load in a non-human mammal that suffers or has suffered from leishmaniasis disease, the method comprising the oral administration of an effective amount of oleylphosphocholine to said mammal.

27. Method of preventing leishmaniasis disease relapse in a non-human mammal, the method comprising the oral administration of an effective amount of oleylphosphocholine to said mammal

28. Veterinary (pharmaceutical) dosage form, comprising oleylphosphocholine in the form of a granulate that:
i) comprises a mixture of olelyl phosphocholine, an anti-oxidant, a filler and, optionally, and/or one or more further granulation excipients and
ii) is obtainable by a melt-agglomeration process, preferably a hot-melt extrusion process, or a wet granulation process, preferably a high-shear wet granulation process; and
wherein the dosage form is adapted for providing one or more metered amounts of the granulate comprising the correct dose of oleylphosphocholine.

29. Veterinary (pharmaceutical) dosage form according to claim 28, wherein:
- the antioxidant is selected from the group consisting of alpha tocopherol, vitamin E, alpha tocopherol acetate, ascorbic acid, butylated hydroxyanisole (BHA), butylated hydroxytoluene (BHT), tert-butylhydroquinone (TBHQ), propyl gallate and ethoxyquin and mixtures thereof, most preferably from alpha tocopherol and Vitamin E; and
- the filler is selected from the group consisting of calcium carbonate, calcium phosphate (dibasic), calcium phosphate (tribasic), calcium sulphate, cellulose, microcrystalline cellulose, microcrystalline silicified cellulose, powdered cellulose, dextrates, dextrose, fructose, lactitol, lactose monohydrate, magnesium carbonate, maltitol, maltodextrin, maltose, mannitol, sodium chloride, sorbitol, starch, pregelatinized starch, sucrose, compressible sugar, sugar spheres, talc, xylitol, silicon dioxide, and mixtures thereof.
